# EUROPÄISCHE PATENTSCHRIFT

(11) **EP 2 842 916 B1**
(45) Veröffentlichungstag und Bekanntmachung des Hinweises auf die Patenterteilung: **23.12.2015**
(21) Anmeldenummer: 14181847.6
(22) Anmeldetag: 21.08.2014
(51) Int. Cl.: C03B 23/055, B29C 53/20, B29K 105/00, C03B 23/045, C03B 23/049, C12M 1/00, G01B 11/04, G01B 11/08, B29C 53/08, C12M 1/12, B29K 101/12, G01B 11/12, C03B 23/09

(54) **VERFAHREN UND VORRICHTUNG ZUR HERSTELLUNG EINES ROHRS MIT EINEM ABSCHNITTSWEISE VON EINER KREISFORM ABWEICHENDEN PROFIL UND KREISFÖRMIGEN ENDABSCHNITTEN.**
METHOD AND DEVICE FOR MANUFACTURING A TUBE, PART OF WHICH HAS A NON-CIRCULAR PROFILE AND CIRCULAR END SECTIONS.
PROCÉDÉ ET DISPOSITIF DE FABRICATION D'UN TUBE AYANT UN PROFIL DÉVIANT DE LA FORME CIRCULAIRE SELON LES SECTIONS ET DOTÉ DE SECTIONS TERMINALES CIRCULAIRES.

(30) Priorität: 30.08.2013 DE 102013109454
(43) Veröffentlichungstag der Anmeldung: 04.03.2015
(73) Patentinhaber: Schott AG, 55122 Mainz (DE)
(72) Erfinder: Schultz, Nikolaus, Dr., 55270 Essenheim (DE); Katsikis, Nikolaos, Dr.-Ing., 95652 Waldsassen (DE); Trinks, Volker, Dr., 95666 Mitterteich (DE); Männl, Reinhard, 95666 Mitterteich (DE)
(74) Vertreter: 2K Patentanwälte Blasberg Kewitz & Reichel

(56) Entgegenhaltungen:
- DE-B3-102006 015 223
- JP-A- 2006 004 660
- JP-A- 2006 315 919

## Beschreibung

### GEBIET DER ERFINDUNG

Die vorliegende Erfindung betrifft allgemein die Herstellung von Rohren aus einem transparenten Glas, Kunststoff oder Kunststoff-Verbundmaterial mit einem von der Kreisform abweichenden Profil mit hoher Präzision, beispielsweise als Ovalrohr, und betrifft insbesondere die Herstellung eines Rohrs mit einem abschnittsweise von einer Kreisform abweichenden Profil und kreisförmigen Endabschnitten.

### HINTERGRUND DER ERFINDUNG

Üblicherweise weisen Rohre ein gleichbleibendes Profil über ihre gesamte Länge auf. Aus dem Stand der Technik sind beispielsweise Glasrohre mit einem kreisförmigen Profil für Anwendungen als Pharma-Packmittel, für Beleuchtungszwecke oder für die Solarthermie bekannt. Ovale Glasrohre, die ein von der Kreisform abweichendes Profil aufweisen, werden von der Anmelderin unter den Marken Conturax^{®} und Conturax Pro^{®} vertrieben. Das Profil ist dabei gleichbleibend über die gesamte Länge der Glasrohre. Ovale Rohre werden auch für Fluoreszenzlampen verwendet (vgl. JP 61224257 A2).

Rohre mit von der Kreisform abweichendem Profil aus Kunststoff oder Kunststoff-Verbundmaterialien finden beispielsweise in Fussbodenheizungen Anwendung.

DE 545449 A offenbart eine Vorrichtung zum Umformen von Glasrohren zu Glasrohren mit einem nicht-kreisrunden Profil, wobei die Glasrohre im erhitzten Zustand durch einen Walzenspalt gefördert werden. DE 1007962 A offenbart ein entsprechendes Verfahren zum Umformen von Glasrohren zu dünnen Isolierbändern oder Isolierschichten. DE 102006015223 B3 der Anmelderin offenbart ein Verfahren und eine Vorrichtung zum Herstellen eines Glasrohrs mit einem von der Kreisform abweichenden Profil. Dabei durchläuft das Ausgangsglasrohr mit dem Ausgangsprofil (Preform) zunächst eine Heißzone unter Ausbildung einer Ziehzwiebel und anschließend einen von einem Walzenpaar ausgebildeten Walzenspalt, in welchem die Ziehzwiebel zu einem Glasrohr mit einem anderen Profil umgeformt wird. Die vorgenannten Glasrohre weisen stets ein gleichbleibendes Profil über ihre gesamte Länge auf.

JP 2006315919 A und JP 2006004660 A2 offenbaren Glasrohre für Kaltkathoden-Fluoreszenzlampen (CCFL) mit Abschnitten mit einem nicht-kreisrunden Profil, die sich mit Abschnitten mit einem kreisförmigen Profil abwechseln. Hierzu wird ein Glasrohr in einem heißen, plastisch verformbaren Zustand abschnittsweise umgeformt, teilweise auch umgebogen. Die Glasrohre sind an den Enden abgeschmolzen, nicht jedoch offen ausgebildet und können somit nicht mit Anschlussmitteln oder benachbarten Glasrohren verbunden werden.

### ZUSAMMENFASSUNG DER ERFINDUNG

Aufgabe der vorliegenden Erfindung ist es, ein Verfahren sowie eine Vorrichtung zur Herstellung von Rohren aus Glas, Kunststoff oder einem Kunststoff-Verbundmaterial mit einem abschnittsweise von der Kreisform abweichenden Profil bereitzustellen, die sich geeigneter und zuverlässiger mit Anschlussmitteln oder benachbarten Rohren verbinden lassen.

Diese Aufgaben werden durch ein Verfahren nach Anspruch 1 und durch eine Vorrichtung nach Anspruch 12 gelöst. Weitere vorteilhafte Ausführungsformen sind Gegenstand der rückbezogenen Unteransprüche.

Gemäß der vorliegenden Erfindung wird ein Verfahren zur Herstellung eines Rohrs mit einem abschnittsweise von einer Kreisform abweichenden Profil durch Umformen mit den folgenden Schritten bereitgestellt: a) Bereitstellen eines Rohrs, das ein kreisförmiges Ausgangsprofil aufweist; b) Fördern des Rohrs in einem plastisch verformbaren Zustand durch einen Walzenspalt, der von Quetschwalzen ausgebildet wird und eine erste Spaltbreite aufweist, die größer oder gleich einer Außenabmessung des Ausgangsprofils ist; c) Verstellen der Quetschwalzen, um eine zweite Spaltbreite des Walzenspalts einzustellen, die kleiner ist als die Außenabmessung des Ausgangsprofils, und Umformen des Ausgangsprofils in dem plastisch verformbaren Zustand zu dem von der Kreisform abweichenden Profil; und d)Verstellen der Quetschwalzen, um eine dritte Spaltbreite des Walzenspalts einzustellen, die größer oder gleich der Außenabmessung des Ausgangsprofils ist, und Abtrennen des Rohrs in einem Bereich mit kreisförmigem Profil; sodass Endabschnitte des Rohrs jeweils ein kreisförmiges Profil aufweisen. Unter dem Begriff "kreisförmiges Profil" ist dabei im Sinne der vorliegenden Erfindung insbesondere gemeint, dass das Profil exakt kreisrund ist bzw. allenfalls im Rahmen der üblichen Herstellungstoleranzen von einem kreisrunden Profil abweicht.

Durch die plastische Verformung in dem Schritt c) wird das den Walzenspalt durchlaufende Rohr in einem plastischen Zustand auf das gewünschte von der Kreisform abweichende Profil umgeformt, beispielsweise zu einem ovalen oder elliptischen Rohr mit einer Hauptachse und einer dazu senkrechten Nebenachse. Die Länge der Nebenachse bzw. die minimale Außenabmessung des Rohrs nach der Umformung kann dabei präzise durch die Breite des Walzenspalts, den das Rohr durchläuft, eingestellt werden. Dieses von der Kreisform abweichende Profil kann sich über einen längeren Abschnitt mit konstantem Profil erstrecken, beispielsweise in einem zentralen Abschnitt des Rohrs, kann jedoch durchaus variieren.

Da in den Schritten b) und d) das in den Walzenspalt einlaufende mit einem kreisrunden Profil Rohr nicht umgeformt wird, weil die Breite des Walzenspalts größer oder gleich einer maximalen Außenabmessung des einlaufenden Rohrs ist, hat das Rohr in den beiden Endabschnitten ein kreisförmiges, idealerweise kreisrundes Profil. Deshalb steht zur Verbindung mit Anschlussmitteln oder benachbarten Rohren, die ebenfalls Endabschnitte mit kreisrundem Profil aufweisen, eine Vielzahl bewährter Rohrverbindungstechnologien zur Verfügung, die für eine präzise und zuverlässige Verbindung in einfacher Weise sorgen können. Hierzu braucht stets nur dafür gesorgt werden, dass die Rohre nur in den Endabschnitten mit kreisförmigem Profil abgelängt werden, nicht jedoch in denjenigen Abschnitten, die ein nicht-kreisrundes Profil aufweisen. Dies gilt insbesondere für die Verwendung von Glasrohren in der Solarthermie oder in Photobioreaktoren.

Für die Ausbildung von Rohren mit kreisförmigem Profil sowie für die Umformung derartiger Rohre in Rohre mit einem von der Kreisform abweichenden Profil mit hoher Präzision sowie geringen Kosten stehen aus dem Stand der Technik bewährte Verfahren und Vorrichtungen zur Verfügung. Das Rohr kann dabei mittels einer Abzieheinrichtung durch den Walzenspalt gezogen werden. Ergänzend oder alternativ zu der Abzieheinrichtung können die Quetschwalzen, welche den Walzenspalt ausbilden, auch aktiv angetrieben werden, um das Rohr ausschließlich oder ergänzend anzutreiben.

Gemäß einer weiteren Ausführungsform erfolgt die Umformung dabei so, dass eine Umfangslänge des von der Kreisform abweichenden Profils und die Umfangslängen der Endabschnitte des Rohrs mit dem kreisförmigen Profil gleich sind

Gemäß einer weiteren Ausführungsform wird eine geförderte Rohrlänge des Rohrs gemessen, wobei auf Grundlage eines Messwerts für die jeweils geförderte Rohrlänge des Rohrs eine axiale Länge des von der Kreisform abweichenden Profils und/oder eine axiale Länge von Übergangsbereichen zwischen den Endabschnitten des Rohrs und dem von der Kreisform abweichenden Profil eingestellt wird. Damit können die Profile dieser Übergangsbereiche sowie deren Variation in axialer Richtung präzise vorgegeben werden, wobei mit dem Messwert für die geförderte Rohrlänge ein zuverlässiger Referenzwert zur Verfügung steht.

Gemäß einer weiteren Ausführungsform erfolgt das Verstellen der Quetschwalzen in den Schritten c) und d) zu diesem Zweck entsprechend einer vorbestimmten Bewegungsfunktion, um die Übergangsbereiche zwischen den Endabschnitten des Rohrs und dem von der Kreisform abweichenden Profil mit Profilen entsprechend der vorbestimmten Bewegungsfunktion auszubilden. Hierzu kann die Breite des Walzenspalts entsprechend einer linearen Funktion verstellt werden. Grundsätzlich können zu diesem Zweck jedoch auch nicht-lineare Funktionen verwendet werden, wobei stetige Funktionen bevorzugt werden.

Gemäß einer weiteren Ausführungsform erfolgt das Umformen des Ausgangsprofils zu dem von der Kreisform abweichenden Profil im Schritt c) in einem heißen plastisch verformbaren Zustand. Dies gilt insbesondere für das Umformen von Rohren aus Glas bei hohen Temperaturen oberhalb des Erweichungspunkts des jeweiligen Glases oder auch für das Umformen von Rohren aus Kunststoffen bei geeigneten Temperaturen. Die Spaltbreite des Walzenspalts wird während der Umformung dabei präzise vorgegeben und aufrechterhalten, um die gewünschte Genauigkeit in der kleinen Achse des Rohrs zu erreichen. Die durch Glasmengen- oder Kunststoffschwankungen und dergleichen hervorgerufenen Schwankungen vor der Umformung werden dabei an den (häufig nicht so wichtigen) großen (ungequetschten) Außendurchmesser des Glasrohrs oder Kunststoffrohrs "weitergegeben". In der Erstreckungsrichtung des Walzenspalts können die Dimensionen somit sehr präzise vorgegeben werden.

Gemäß einer weiteren Ausführungsform wird die Lage von zumindest einer der Quetschwalzen permanent so verändert, dass eine Kontaktfläche zwischen der jeweiligen Quetschwalze und dem heißen Rohrkörper permanent variiert wird bzw. sich ändert. Durch diese überraschend einfache Maßnahme können erfindungsgemäß insbesondere Glasrohre mit abschnittsweise von einer Kreisform abweichendem Profil mit noch höherer Präzision hergestellt werden. Denn die Kontaktfläche zwischen dem heißen, zu verformenden Rohr und den Quetschwalzen ändert sich bei dieser Ausführungsform ständig. Dadurch werden diverse Probleme gegenüber herkömmlichen Quetschböcken mit einer (quasi-)fixen Position des Quetschwalzen verhindert. Quasi-fix bedeutet in diesem Zusammenhang insbesondere, dass die Kontaktposition der Walzen mit dem heißen, zu verformenden Rohr nicht veränderlich ist. Verletzungen in der Oberfläche des zu verformenden Rohrs durch zu heiße Walzen, kleine Partikel oder dergleichen können aus der Oberfläche des zu verformenden Rohrs beim Quetschen herausgerissen werden und stören somit die weitere Umformung nicht weiter. Unterschiedliche Temperaturen auf der Oberfläche der Quetschwalzen, die herkömmlich zu unterschiedlichen Spaltmaßen führen und somit zu schlechten Toleranzen aufgrund einer Veränderung der Kontaktfläche führten, können so ebenfalls verringert werden. Ferner kann eine unerwünschte Überhitzung der Kontaktfläche zwischen dem zu verformenden Rohr und Quetschwalzen vermieden werden, die herkömmlich häufig zu deutlich sichtbaren unerwünschten "Hitzespuren" auf der Außenoberfläche des zu verformenden Rohrkörpers geführt hatten. Die Quetschwalzen können nach dieser Ausführungsform auch länger verwendet werden, insbesondere ist ein maschinelles Polieren der Quetschwalzen vor deren Wiederverwendung, wenn überhaupt, dann jedenfalls wesentlich seltener erforderlich. Mit dem erfindungsgemäßen Verfahren lässt sich auch eine ruhigere und gleichmäßigere Außenoberfläche des Rohrkörpers erzielen.

Auf Grund der kontinuierlichen Verstellung können die Quetschwalzen grundsätzlich auch ohne Kühlung betrieben werden, was bei einem herkömmlichen Verfahren häufig zu zusätzlichen Problemen mit der Maßhaltigkeit des gequetschten Rohrkörpers geführt hatte.

Bevorzugt wird der Walzenspalt von zwei einander gegenüberliegenden Quetschwalzen ausgebildet, die parallel zueinander verlaufend verschieblich gelagert sind. Wenngleich es grundsätzlich ausreichend sein kann, wenn die Lage von nur einer dieser beiden Quetschwalzen permanent (axial) verstellt wird, wird gemäß einer weiteren Ausführungsform bevorzugt die Lage von beiden Quetschwalzen permanent in Ihrer Lage (axial) verstellt werden. Bevorzugt werden diese beiden Quetschwalzen gemeinsam und synchron zueinander axial verstellt.

Um die vorgenannte permanente Verstellung der jeweiligen Quetschwalze zu erzielen, ist der jeweiligen Quetschwalze eine Verstelleinrichtung oder Antrieb zugeordnet, der mit der jeweiligen Quetschwalze gekoppelt ist und diese so verstellt, dass eine Kontaktfläche zwischen der jeweiligen Quetschwalze und dem heißen Rohrkörper permanent variiert wird bzw. sich ändert.

Gemäß einer weiteren Ausführungsform kann die vorgenannte permanente Verstellung der jeweiligen Quetschwalze unter Beibehaltung der aktuellen Spaltbreite zwischen den den Walzenspalt ausbildenden Quetschwalzen erfolgen. Dies soll nicht ausschließen, dass ergänzend auch die Breite des Walzenspalts zwischen den diesen ausbildenden Quetschwalzen gesteuert oder geregelt wird, beispielsweise um eine konstante Außenabmessung des Rohrkörpers oder eine konstante Wandstärke desselben zu erzielen. Die Verstellung der jeweiligen Quetschwalze und eine solche Steuerung oder Regelung der Breite des Walzenspalts sind jedoch bevorzugt nicht miteinander gekoppelt und erfolgen zu einem völlig anderen Zweck. Dies kann sich beispielsweise auch in deutlich unterschiedlichen Zeitskalen ausdrücken, auf denen die permanente Verstellung der Position der jeweiligen Quetschwalze und die Steuerung oder Regelung der Breite des Walzenspalts erfolgen.

Gemäß einer weiteren Ausführungsform wird die Lage der zumindest einen Quetschwalze bzw. bevorzugt der beiden Quetschwalzen durch permanentes axiales Verstellen der Quetschwalzen entsprechend einer vorbestimmten Bewegungsfunktion verändert wird. Diese Bewegungsfunktion wird zweckmäßig überwiegend stetig ausgeführt und kann beispielsweise entsprechend einem Sägezahn- oder Sinussignal ausgeführt werden, wobei darauf zu achten ist, dass ein allzu langes Verharren der Quetschwalze(n) an der gleichen Position zu vermeiden ist, weil dann die Nachteile von herkömmlichen Umformverfahren wieder auftreten würden.

Gemäß einer bevorzugten weiteren Ausführungsform wird die Bewegungsfunktion als zyklische Hin- und Herbewegung der jeweiligen Quetschwalze oder bevorzugt der beiden den Walzenspalt ausbildenden Quetschwalzen in deren axialer Richtung ausgeführt. Bevorzugt ist diese Hin- und Herbewegung zeitlich symmetrisch.

Gemäß einer weiteren Ausführungsform wird die Bewegungsfunktion in diskreten Schritten gleicher Schrittgröße ausgeführt, was die Umsetzung der Bewegungsfunktion mittels handelsüblicher Synchronmotoren oder Schrittmotoren oder dergleichen begünstigt.

Gemäß einer weiteren Ausführungsform wird eine Drehbewegung der den Walzenspalt ausbildenden Quetschwalzen jeweils separat angetrieben. Dies ermöglicht eine hochgenaue Einstellung des Profils des Rohrkörpers nach der Umformung, beispielsweise der ovalen Form eines herzustellenden Ovalrohrs in den von den Endabschnitten verschiedenen Abschnitten. Die Quetschwalzen werden dabei bevorzugt auf die Geschwindigkeit synchronisiert, mit der der Rohrkörper den Walzenspalt durchläuft.

Gemäß einer weiteren Ausführungsform wird die Drehbewegung der den Walzenspalt ausbildenden Quetschwalzen jeweils synchron oder mit einem vorbestimmten konstanten Offset angetrieben, sodass man die Ziehgeschwindigkeit des Rohrkörpers durch den Walzenspalt ändern und die Geschwindigkeit der Quetschwalzen automatisch mit ändern kann.

Gemäß einer weiteren Ausführungsform wird eine Außenabmessung und/oder Innenabmessung des anderen Profils stromabwärts des Walzenspalts erfasst und ein die Umformung des heißen, plastisch verformbaren Rohrkörpers zu einem Rohrkörper mit dem anderen Profil betreffender Parameter in Entsprechung zu der erfassten Außenabmessung und/oder Innenabmessung des anderen Profils gesteuert oder geregelt, um die Außenabmessung und/oder Innenabmessung des anderen Profils konstant zu halten.

Hierzu kann beispielsweise der Außendurchmesser und/oder der Innendurchmesser mittels eines optischen Messverfahrens, das stromabwärts von dem Walzenspalt und zweckmäßig noch vor einer Ziehmaschine ausgeführt wird, die den Rohrkörper durch den Walzenspalt abzieht, permanent gemessen und überwacht werden. Im Falle einer unerwünschten Änderung kann dann der relevante Parameter nachgesteuert oder -geregelt werden, um eine noch höhere Präzision zu erzielen.

Gemäß einer weiteren Ausuhrungsform werden die den Walzenspalt ausbildenden Quetschwalzen automatisch vermessen, insbesondere deren Rundlaufgenauigkeit und/oder Außendurchmesser, und diese mittels einer Regelung so zueinander positioniert, dass die Rundlauffehler und/oder Außendurchmesservariation, bezogen auf den Spaltabstand ein Minimum ergeben.

Gemäß einer weiteren Ausführungsform ist der Parameter die Spaltbreite des Walzenspalts, eine jeweilige Drehzahl oder Drehzahldifferenz der den Walzenspalt ausbildenden Quetschwalzen oder ein an einen Innenraum des als Rohr ausgebildeten plastisch verformbaren Rohrkörpers angelegter Überdruck.

Gemäß einer weiteren Ausführungsform wird der Rohrkörper entsprechend der stromabwärts des Walzenspalts erfassten Außenabmessung und/oder Innenabmessung markiert und/oder sortiert. Somit können Chargen mit vorbestimmten Toleranzen einfacher erstellt werden.

Um die vorgenannte Verstellung der Quetschwalzen zu erzielen, ist den Quetschwalzen und bevorzugt jeder der Quetschwalzen zumindest eine Verstelleinrichtung zugeordnet. Gemäß einer bevorzugten Ausführungsform umfasst die Verstelleinrichtung einen Verschiebetisch und einen Verstellmotor zum Verstellen des Verschiebetisches, wobei die Quetschwalzen auf dem Verschiebetisch gelagert sind, der Verschiebetisch in Axialrichtung der Quetschwalzen verschieblich gelagert ist und der Verstellmotor mit dem Verschiebetisch gekoppelt ist, um das axiale Verstellen der zumindest einen Quetschwalze bzw. der beiden den Walzenspalt ausbildenden Quetschwalzen durch Verschieben des Verschiebetisches zu bewirken.

Gemäß einer weiteren Ausführungsform laufen die Achsen der Quetschwalzen, welche den Walzenspalt ausbilden, V-förmig aufeinander zu, was eine größere Variabilität bei der Ausbildung des Profils des Rohrs zwischen den kreisförmigen Endabschnitten ermöglicht. Insbesondere lassen sich mit einer V-förmig aufeinander zulaufenden Orientierung der Quetschwalzen Rohr mit tropfenförmigen zentralen Abschnitten realisieren.

Gemäß einer weiteren Ausführungsform weisen die Quetschwalzen, welche den Walzenspalt ausbilden, ein nicht-kreisförmiges Profil auf, insbesondere ein ovalen oder mehreckiges Profil, sodass das Profil des Rohrs zwischen den kreisförmigen Endabschnitten, in Axialrichtung betrachtet, variiert, beispielsweise gewellt ist oder Einbauchungen und Erhebungen aufweist. Auf diese einfache Weise können insbesondere regelmäßig wiederkehrende Konturen auf dem Außenumfang des zentralen Abschnitts zwischen den kreisförmigen Endabschnitten ausgebildet werden.

Gemäß einer weiteren Ausführungsform weisen die Quetschwalzen, welche den Walzenspalt ausbilden, ein rotationssymmetrisches Profil mit von der Linienform abweichender Kontur auf, also in Axialrichtung betrachtet eine von einer Zylinderform abweichende Kontur. Dadurch lässt sich erfindungsgemäß eine noch größere Variabilität bei der Ausbildung des Profils des Rohrs zwischen den kreisförmigen Endabschnitten realisieren.

Gemäß einer weiteren Ausführungsform bilden die Quetschwalzen einen spiegelsymmetrischen Walzenspalt aus, wobei die Kontur der jeweiligen Quetschwalze zumindest eine Vertiefung oder zumindest einen Vorsprung aufweist. Auf diese Weise lassen sich Rohre mit nahezu beliebig profilierten zentralen Abschnitten zwischen den kreisförmigen Endabschnitten realisieren, beispielsweise mit Einbauchungen. Solchermaßen hergestellte Rohre können in ihren zentralen Abschnitten insbesondere eine gewisse Symmetrie einhalten, beispielsweise axialsymmetrisch oder spiegelsymmetrisch ausgebildet sein.

Gemäß einer weiteren Ausführungsform sind die Quetschwalzen, welche den Walzenspalt ausbilden, aktiv angetrieben. Dadurch kann beispielsweise vermieden werden, dass sich das Rohrmaterial im wärmeweichen Zustand während der Umformung vor dem Walzenspalt staut, beispielsweise wenn Rohre mit einem zentralen Abschnitt zwischen den kreisförmigen Endabschitten mit vergleichsweise hoher Ovalität ausgebildet werden sollen. Ein solcher aktiver Antrieb der Quetschwalzen kann gemäß einer weiteren Ausführungsform gar eine vorgesehene Abziehmaschine ersetzen, die üblicherweise vorgesehen ist, um das Rohr aus einer stromaufwärts befindlichen Herstellungsanlage und/oder durch den Walzenspalt abzuziehen, beispielsweise bei der Herstellung von Glasrohren. Der aktive Antrieb der den Walzenspalt ausbildenden Quetschwalzen kann dabei geeignet gesteuert oder geregelt werden, um im Walzenspalt gleichbleibende Verhältnisse und vorherrschende Kräfte zu realisieren. Der aktive Antrieb der den Walzenspalt ausbildenden Quetschwalzen kann insbesondere auch zu einer geringeren Belastung auf das Rohrmaterial beim Transport durch den Walzenspalt führen, insbesondere zu weniger Materialspannungen beim Abkühlen des Rohres stromabwärts des Walzenspalts, und kann insbesondere auch einen Abriss des Rohrmaterials verhindern, der nach dem Stand der Technik immer dann aufzutreten droht, wenn das Rohrmaterial zu fest durch den Walzenspalt gezogen wird, beispielsweise bei der Herstellung von Glasrohren.

Gemäß einer weiteren Ausführungsform werden die Quetschwalzen, welche den Walzenspalt ausbilden, zusätzlich geheizt. Die kann eine Verformung des Rohrmaterials beim Transport durch den Walzenspalt erleichtern, weil das Rohrmaterial gezielt lokal erwärmt oder in der Temperatur geeignet eingestellt werden kann, um in diesem Bereich eine geeignete Verformbarkeit zu ermöglichen. Insbesondere kann somit auch der auf das Rohrmaterial im Walzenspalt ausgeübte Druck weiter reduziert werden, was erfindungsgemäß die Herstellung von noch spannungsärmeren Rohren ermöglicht. Eine Beheizung der Quetschwalzen, welche den Walzenspalt ausbilden, kann auch eine größere Variabilität beim Design der Herstellungsanlage und bessere Toleranzen ermöglichen. Denn während herkömmlich die Quetschwalzen dort positioniert werden mussten, wo das sich abkühlende Rohrmaterial sich noch in einem geeigneten wärmeweichen Zustand befunden hat, so können erfindungsgemäß die Quetschwalzen auch an einem anderen Ort, insbesondere stromabwärts vom herkömmlich erforderlichen Ort, positioniert werden, da durch die Beheizung der Quetschwalzen das Rohrmaterial erneut in einen geeignet wärmeweichen Zustand zurück versetzt werden kann.

Eine solche Beheizbarkeit ist insbesondere dann von Vorteil, wenn die Quetschwalzen ein nicht-kreisförmiges Profil aufweisen, weil so plötzliche Stauungen von Rohrmaterial im Walzenspalt im Falle einer örtlichen Änderung des Profils des Walzenspalts durch Vorgabe geeigneter (auch zeitabhängiger) Temperaturen im Walzenspalt vermieden werden können.

Gemäß einer weiteren Ausführungsform werden die Quetschwalzen axial verstellt und/oder deren Anstellwinkel verändert. Dadurch lässt sich erfindungsgemäß eine noch größere Variabilität bei der Ausbildung des Profils des Rohrs zwischen den kreisförmigen Endabschnitten realisieren.

Ein weiterer Gesichtspunkt der vorliegenden Erfindung betrifft eine entsprechend ausgelegte Vorrichtung zur Herstellung eines Rohrs mit einem abschnittsweise von einer Kreisform abweichenden Profil durch Umformen, wie nach dem Patentanspruch 12 beansprucht.

Gemäß einer weiteren Ausführungsform kann eine solche Vorrichtung weiterhin umfassen eine Messeinrichtung, um eine geförderte Rohrlänge des Rohrs zu messen, wobei die Steuereinrichtung die Verstelleinrichtung ferner auf Grundlage eines Messwerts für die jeweils geförderte Rohrlänge des Rohrs so steuert, dass eine axiale Länge des von der Kreisform abweichenden Profils und/oder eine axiale Länge von Übergangsbereichen zwischen den Endabschnitten des Rohrs und dem von der Kreisform abweichenden Profil eingestellt wird.

Solchermaßen hergestellte Rohre, insbesondere aus einem transparenten Glas oder Kunststoff, sind insbesondere geeignet für sämtliche Anwendungen, bei denen Rohre mit einem von der idealen Kreisform abweichenden Profil notwendig sind oder jedenfalls Vorteile bieten, jedoch in einfacher und kostengünstiger Weise mit Anschlussmitteln, beispielsweise rohrförmigen Zu- oder Abläufen, oder benachbarten Rohren verbunden werden sollen.

Eine bevorzugte erste Verwendung betrifft die Verwendung eines transparenten Rohrs zur Bestrahlung von Mikroorganismen mittels einer künstlichen Lichtquelle oder bevorzugter mittels natürlichem Sonnenlicht, insbesondere in einem Photobioreaktor, wobei das Rohr zumindest einen zentralen Abschnitt mit einem von einer Kreisform abweichenden Profil und zwei Endabschnitte mit jeweils einem kreisförmigen Profil aufweist, wobei das Rohr über die Endabschnitte mit einem Einlass und Auslass oder mit benachbarten Rohren verbunden ist und in einem Innenvolumen des Rohrs die zu bestrahlenden Mikroorganismen angeordnet sind, bei welcher Verwendung der zumindest eine zentrale Abschnitt des Rohrs mit seiner breiteren Seite der künstlichen Lichtquelle oder dem Sonnenlicht bzw. der Richtung des Sonnenlichts zugewandt angeordnet wird. Aufgrund der geringeren Abmessung des Rohrs in der Einfallsrichtung der Bestrahlung, beispielsweise von Licht, können in das Rohr eingebrachte Mikroorganismen intensiver und effizienter bestrahlt werden, was erhebliche Kostenvorteile bei Verwendung der erfindungsgemäß hergestellten Rohre ermöglicht.

Eine bevorzugte zweite Verwendung betrifft die Verwendung eines transparenten Rohrs zur Erwärmung eines Fluids mittels Sonnenlicht, wobei das Rohr zumindest einen zentralen Abschnitt mit einem von einer Kreisform abweichenden Profil und zwei Endabschnitte mit jeweils einem kreisförmigen Profil aufweist, wobei das Rohr über die Endabschnitte mit einem Einlass und Auslass oder mit benachbarten Rohren verbunden ist und ein Innenvolumen des Rohrs von dem zu erwärmenden Fluid, beispielsweise einem Sonnenlicht absorbierenden Öl, durchströmt ist, bei welcher Verwendung der zumindest eine zentrale Abschnitt des Rohrs mit seiner breiteren Seite dem Sonnenlicht zugewandt angeordnet wird. Aufgrund der geringeren Abmessung des Rohrs in der Einfallsrichtung des Sonnenlichts kann das das Rohr durchströmende Fluid intensiver und effizienter bestrahlt werden, was erhebliche Kostenvorteile bei Verwendung der erfindungsgemäß hergestellten Rohre ermöglicht.

Ein weiterer Gesichtspunkt der vorliegenden Anmeldung betrifft ein Rohr, insbesondere aus einem transparenten Material, ganz besonders bevorzugt als Glasrohr ausgebildet, mit zumindest einem zentralen Abschnitt, der ein von einer Kreisform abweichendes Profil aufweist, und zwei Endabschnitten, die jeweils ein kreisförmiges Profil aufweisen, wobei das Rohr aus einem Material ausgebildet ist, das in eine erwärmten Zustand plastisch verformbar ist. Weitere bevorzugte Profilverläufe derartiger Rohre werden nachfolgend ausführlicher beschrieben.

### FIGURENÜBERSICHT

Nachfolgend wird die Erfindung in beispielhafter Weise und unter Bezugnahme auf die beigefügten Zeichnungen beschrieben werden, woraus sich weitere Merkmale, Vorteile und zu lösende Aufgaben ergeben werden. Es zeigen:
- Fig. 1: in einer schematischen Darstellung eine Glasrohrherstellungsanlage gemäß der vorliegenden Erfindung;
- Fig. 2: in einer Vorderansicht eine Vorrichtung zum Umformen gemäß einer ersten Ausführungsform der vorliegenden Erfindung;
- Fig. 3: die Vorrichtung nach der Fig. 2 in einer Seitenansicht;
- Fig. 4a: die Vorrichtung nach der Fig. 2 in einer Draufsicht ohne eine Abdeckung, die einer Abschirmung des Umformbereichs dient;
- Fig. 4b: die Vorrichtung nach der Fig. 2 in einer Draufsicht mit der Abdeckung, die einer Abschirmung des Umformbereichs dient;
- Fig. 5a: in einer perspektivischen Vorderansicht eine Vorrichtung zum Umformen gemäß einer zweiten Ausführungsform der vorliegenden Erfindung ohne eine Abdeckung, die einer Abschirmung des Umformbereichs dient;
- Fig. 5b: in einer perspektivischen Vorderansicht die Vorrichtung nach der Fig. 5a mit der Abdeckung, die einer Abschirmung des Umformbereichs dient;
- Fig. 6: in einer perspektivischen Draufsicht den oberen Bereich der Vorrichtung nach der Fig. 2 mit den Quetschwalzen;
- Fig. 7a bis 7e: Beispiele für Glasrohre, die mittels einer Vorrichtung nach der vorliegenden Erfindung durch Umformung ausgebildet werden;
- Fig. 8: ein beispielhaftes Flussdiagramm eines Verfahrens gemäß der vorliegenden Erfindung zur Herstellung von einem Rohr mit einem abschnittsweise von einer Kreisform abweichenden Profil und kreisförmigen Endabschnitten;
- Fig. 9a: die Verwendung eines Rohrs gemäß der vorliegenden Anmeldung in einem Photobioreaktor zum Züchten von Mikroorganismen durch Bestrahlung gemäß einer ersten Ausführungsform;
- Fig. 9b: die Verwendung eines Rohrs gemäß der vorliegenden Anmeldung in einem Photobioreaktor zum Züchten von Mikroorganismen durch Bestrahlung gemäß einer weiteren Ausführungsform;
- Fig. 9c: eine weitere Verwendung eines Rohrs gemäß der vorliegenden Anmeldung für Anwendungen in der Solarthermie;
- Fig. 10a: den Bereich eines Walzenspalts, der von zwei Quetschwalzen ausgebildet wird, für ein Verfahren gemäß einer weiteren Ausführungsform der vorliegenden Erfindung;
- Fig. 10b: eine Quetschwalze zur Durchführung des Verfahren gemäß der Fig. 10a;
- Fig. 11a: der Verbindung von zwei Rohren, die nach einem Verfahren gemäß der vorliegenden Erfindung hergestellt sind; und
- Fig. 11b: in einer schematischen Darstellung einen Photobioreaktor zum Züchten von Mikroorganismen durch Bestrahlung, mit Rohren, die nach einem Verfahren gemäß der vorliegenden Erfindung hergestellt sind.

In den Figuren bezeichnen identische Bezugszeichen identische oder im Wesentlichen gleichwirkende Elemente oder Elementgruppen.

### AUSFÜHRLICHE BESCHREIBUNG VON BEVORZUGTEN AUSFÜHRUNGSBEISPIELEN

Nachfolgend wird die Erfindung anhand eines Ausführungsbeispiels zum Umformen von Glasrohren beschrieben. Die vorliegende Erfindung ist jedoch nicht auf das Umformen von Glasrohren beschränkt, sondern kann in gleicher Weise auch für Rohre aus anderen Materialien eingesetzt werden, insbesondere auf das Umformen von Rohren aus Kunststoffen oder Kunststoff-Verbundmaterialien.

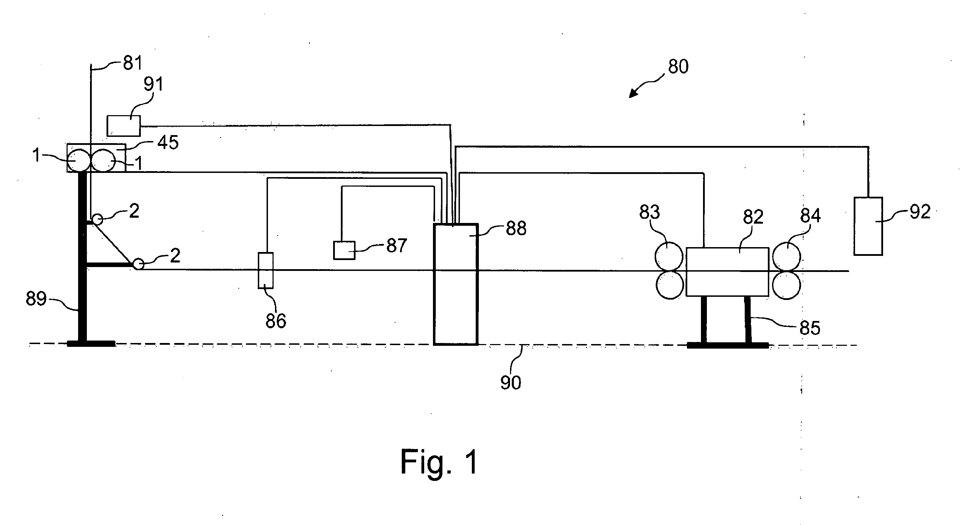

Gemäß der Fig. 1 umfasst die insgesamt mit 80 bezeichnete Anlage ein Gestell 89, an dem zwei Quetschwalzen 1 zum Umformen des in deren Walzenspalt einlaufenden Glasrohrstrangs 81 sowie stromabwärts davon zwei Umlenkrollen 2 gelagert sind, um den umgeformten Glasrohrstrang in Richtung der Ziehmaschine 82 mit den beiden Zugrollenpaaren 83, 84 umzulenken. Ein Gehäusedeckel 45, in welchem die Quetschwalzen 1 angeordnet sind, schirmt den Umformungsbereich im Bereich der Quetschwalzen 1 von der Außenumgebung ab.

Der Außendurchmesser des Glasrohrstrangs 81 wird mit Hilfe eines Messgeräts 86 bevorzugt berührungsfrei und ganz besonders bevorzugt optisch gemessen. Der Innendurchmesser des Glasrohrstrangs 81 wird mit Hilfe eines Messgeräts 87 optisch gemessen, insbesondere mit Hilfe eines Triangulationsverfahrens. Der Messort des Messgeräts 87 befindet sich bevorzugt möglichst nahe an dem Messort des Messgeräts 86. Ferner werden die beiden Quetschwalzen 1 automatisch vermessen, beispielsweise mittels induktiver Wegmessung, und der Rundlauffehler und/oder die Außendurchmesserschwankungen der Quetschwalzen bestimmt. Ferner wird die geförderte Länge des Rohrstrangs 81 mittels eines Messgeräts 91 gemessen, bevorzugt berührungslos.

Wie durch die Verbindungslinien in der Fig. 1 angedeutet, werden die relevanten Komponenten der Anlage 80 von einer Steuerungs- oder Regelungseinrichtung 88 gesteuert oder geregelt, insbesondere von einer CPU basierend auf einer hierzu ausgelegten Software. Damit kann auf einen konstanten Außendurchmesser der Glasrohre gesteuert oder geregelt werden, oder auf eine konstante Wandstärke des Glasrohrs oder so, dass der Rundlauffehler der Quetschwalzen 1 bezogen auf die Breite des von diesen ausgebildeten Walzenspalts ein Minimum ergeben.

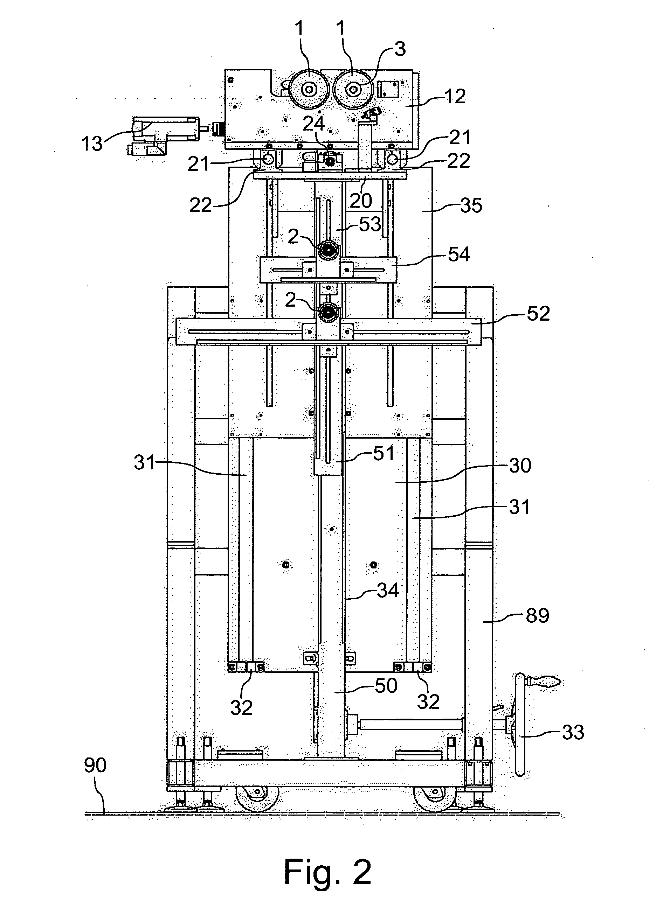

Die Fig. 2 zeigt in einer Vorderansicht eine Vorrichtung zum Umformen gemäß einer ersten Ausführungsform der vorliegenden Erfindung. An einem auf dem Boden 90 abgestützten Maschinengestell 89 ist eine Grundplatte 30 befestigt, an der der Verschiebetisch 35 vertikal verschiebbar gelagert ist, um eine Einstellung der Höhe der Quetschwalzen 1 zu ermöglichen. Diese Höhenverstellung kann manuell mit Hilfe des Handrads 33 erfolgen, aber auch motorisch erfolgen.

An der Grundplatte 30 sind zwei Führungsstangen 31 befestigt, welche den Verschiebetisch vertikal führen. Die Verstellung wird mittels einer Gewindespindel 34 bewerkstelligt, die einerseits in das als Vertikal-Verstelleinrichtung wirkende Handrad 33 und andererseits in den Verschiebetisch 35 eingreift oder damit gekoppelt ist.

Gemäß der Fig. 2 ist die Trägerplatte 20, an welcher die Quetschwalzen (nicht dargestellt) gelagert sind, über einen horizontalen Adapter 38 und einen vertikalen Adapter 37 mit dem Verschiebetisch 35 verbunden. Durch Verstellen des Handrads 33 kann somit die Höhe der Quetschwalzen 1 eingestellt werden. Die Höhe wird dabei so eingestellt, dass entlang des sich in der Abzugsrichtung rasch abkühlenden Glasrohrstrangs ein Temperaturbereich bereitgestellt wird, in welchem der Glasrohrstrang geeignet plastisch ist, um präzise umgeformt werden zu können. Diese Höhe kann einmalig, beispielsweise bei Inbetriebnahme der Anlage, eingestellt werden oder kontinuierlich oder zyklisch mittels der Steuerung oder Regelung 88 nachjustiert werden.

Gemäß der Fig. 2 können die Höhe der Umlenkrollen 2 und deren Stellung in einer Richtung quer zur Abzugsrichtung des Glasrohrstrangs unabhängig voneinander mittels der Höhenverstellungen 51 und 53 sowie mittels der Querverstellungen 52 und 54 präzise eingestellt oder nachjustiert werden.

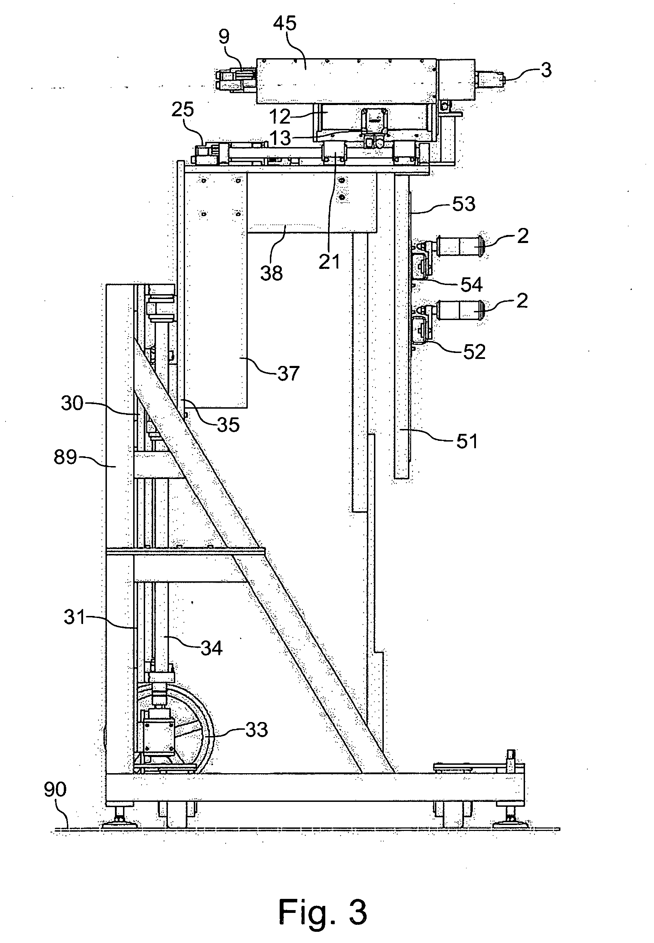

Die Fig. 3 zeigt die Vorrichtung nach der Fig. 2 in einer Seitenansicht.

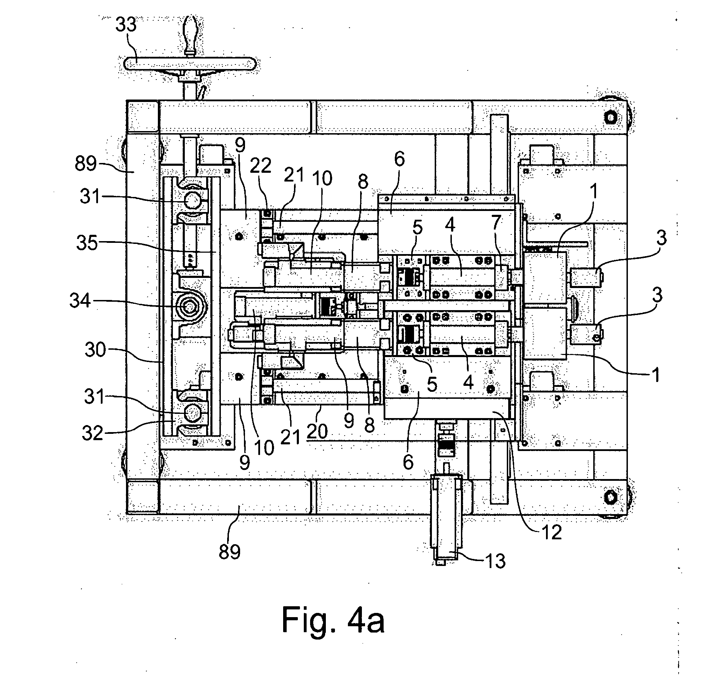

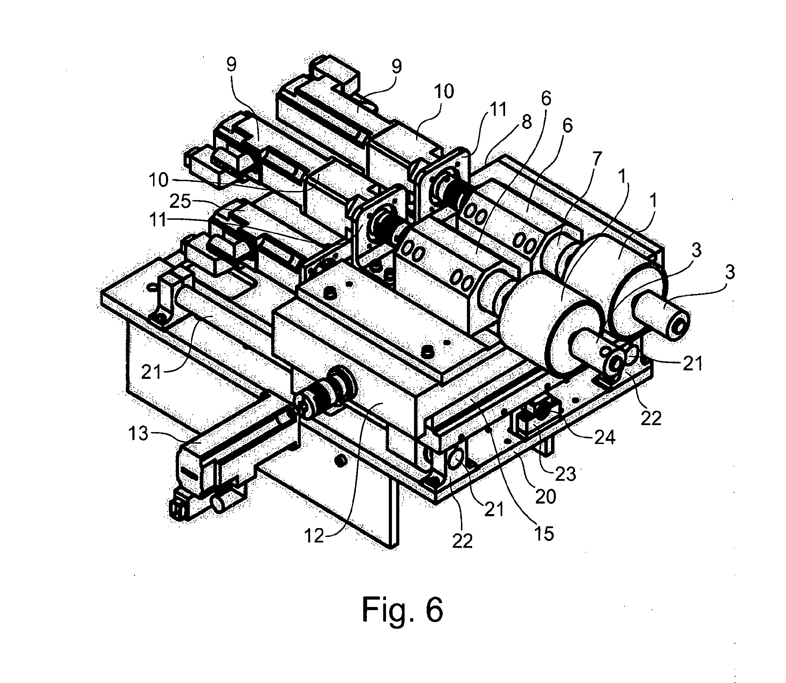

Der weitere Aufbau zur Verstellung der Quetschwalzen 1 ergibt sich am übersichtlichsten aus der Zusammenschau der Draufsicht gemäß der Fig. 4a und der Perspektivansicht gemäß der Fig. 6. Es sei darauf hingewiesen, dass die nachfolgend ausführlich beschriebene axiale Verstellung der Quetschwalzen 1 während der Umformung des Glasrohrstrangs nicht unbedingt notwendig ist und dass die Umformung auch bei axial feststehenden Quetschwalzen 1 grundsätzlich möglich ist. Diese axiale Verstellung der Quetschwalzen 1 während der Umformung des Glasrohrstrangs ist jedoch für die Umformung bei hohen Temperaturen vorteilhaft, also insbesondere bei der Umformung von Glasrohren bei Temperaturen oberhalb der Erweichungstemperatur.

Wie in den Figuren 4a und 6 dargestellt, sind die beiden einen Walzenspalt ausbildenden Quetschwalzen 1 an einem Verschiebetisch 12 gelagert, der die beiden Quetschwalzen 1 trägt. Der Verschiebetisch 12 kann mit Hilfe des Verstellmotors 25 in Axialrichtung der Quetschwalzen 1 verstellt werden, um so die beiden Quetschwalzen gemeinsam axial zu verstellen.

Gemäß einer weiteren Ausführungsform (nicht gezeigt) können die beiden einen Walzenspalt ausbildenden Quetschwalzen 1 an einem zweiteilig ausgebildeten Verschiebetisch 12 gelagert sein, dessen eine Hälfte eine der Quetschwalzen 1 trägt und dessen andere Hälfte die andere der beiden Quetschwalzen 1 trägt. Während die eine Hälfte des Verschiebetisches 12 gemäß dieser Ausführungsform während der Umformung ortsfest gehalten wird, kann die andere Hälfte des Verschiebetisches 12 mit Hilfe des Verstellmotors 25 in Axialrichtung der Quetschwalzen 1 relativ zu der ersten Hälfte des Verschiebetisches 12 verstellt werden.

Zur Führung des Verschiebetisches 12 sind an der Trägerplatte 20 zwei zueinander parallele Führungsstangen 21 an jeweiligen Lagerblöcken 22 gelagert. Auf der Unterseite des Verschiebetisches 12 vorgesehene Gleitelemente (nicht dargestellt), welche in die Führungsstangen 21 eingreifen, führen die axiale Verstellung des Verschiebetisches. Zur Verstellung des Verschiebetisches ist weiterhin eine Verstell-Gewindespindel 24 an der Trägerplatte befestigt, die von dem Verstellmotor 25 drehangetrieben wird und in ein Gegengewinde (nicht dargestellt) auf der Unterseite des Verschiebetisches 12 eingreift.

Der Verstellmotor 25 ist dabei als Synchronmotor ausgebildet, kann jedoch auch als Schrittmotor ausgeführt sein, um eine schrittweise axiale Verstellung zu ermöglichen, wie nachfolgend näher ausgeführt.

Gemäß der Fig. 6 ist dem Verschiebetisch 12 ferner eine quer verlaufende Führungsschiene 15 vorgesehen, die die Verstellung des Verschiebetisches (oder gemäß der vorgenannten alternativen Ausführungsform der einen relativ zu der anderen Hälfte des Verschiebetisches 12) bei der Verstellung der Spaltbreite des Walzenspalts führt. Zur Einstellung der Spaltbreite des Walzenspalts ist ein Verstellmotor 13 vorgesehen, der an der Trägerplatte 20 befestigt ist. Dadurch kann die verstellbare Quetschwalze 1 relativ zu der anderen, ortsfest gelagerten Quetschwalze verstellt werden.

Es sei darauf hingewiesen, dass grundsätzlich auch beiden Quetschwalzen 1 axial verstellt werden können.

Zum Antreiben der Drehbewegung der Quetschwalzen 1 sind die beiden Servomotoren 9 vorgesehen, die über ein jeweiliges Getriebe 10 und eine jeweilige Kupplung 8 mit der zugeordneten Quetschwalze 1 gekoppelt sind. Die Kupplung 8 ist in einem Kupplungsgehäuse aufgenommen, deren vorderes flanschartiges Ende 11 von der Kupplung 8 teilweise durchgriffen wird. Die Kupplung 8 koppelt mit einer Spindelwelle 4, die mittels Lagern 5 / Spindellagern 7 in einem jeweiligen als Gehäuse ausgebildeten Lagerblock 6 gelagert ist. Die Quetschwalzen 1 können an Befestigungsflanschen am vorderen Ende der jeweiligen Spindelwelle 4 befestigt sein.

In das vordere Ende der Quetschwalzen 1 greift eine jeweilige Drehdurchführung 3 ein, die mittels Luft oder eines Fluids, wie beispielsweise Wasser, gekühlt werden kann, um die Quetschwalzen weiter abzukühlen. Es sei jedoch darauf hingewiesen, dass aufgrund der erfindungsgemäß permanent ausgeführten axialen Verstellung der Quetschwalzen 1 während der Umformung des Glasrohrs eine solche Kühlung nicht unbedingt notwendig ist.

Es ist möglich, die Drehgeschwindigkeit der beiden Quetschwalzen 1 separat zu steuern. Weiterhin wird diese Drehgeschwindigkeit hochgenau digital erfasst und angezeigt. Das ist vorteilhaft bei der Einstellung der Form des Ovalrohres.

Es ist möglich die Drehgeschwindigkeit der Quetschwalzen 1 mit der Geschwindigkeit der Ziehmaschine 82 (vgl. Fig. 1) am Zug zu synchronisieren bzw. mit einem festen Offset aneinander zu koppeln. Das heißt, man kann die Ziehgeschwindigkeit des Zuges ändern und die Geschwindigkeit der Quetschwalzen wird automatisch mit geändert.

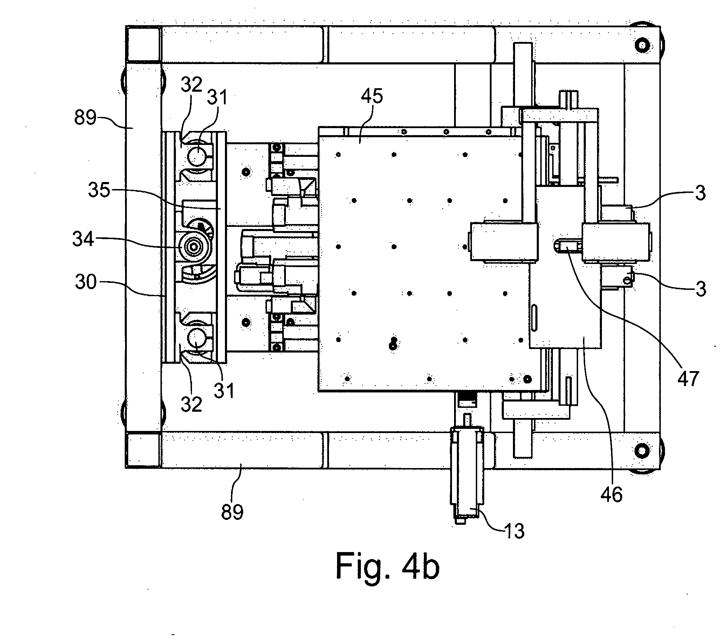

Wie man in der Fig. 4b erkennen kann, sind die Quetschwalzen im Betriebszustand gemeinsam in einem Gehäuse 45 untergebracht und von einer weiteren Abdeckung 46 abgedeckt, in welcher eine Öffnung 47 ausgebildet ist, durch welche der Glasrohrstrang von oben in den Walzenspalt zur Umformung einläuft.

Während der Umformung des Glasrohrstrangs im Walzenspalt wird die Lage von bevorzugt beiden Quetschwalzen 1 gemeinsam durch permanentes axiales Verstellen der Lage der Quetschwalzen so verändert, dass eine Kontaktfläche zwischen der jeweiligen Quetschwalze und dem heißen Glaskörper permanent variiert wird bzw. sich ändert. Bevorzugt erfolgt dieses permanente axiale Verstellen der Lage der Quetschwalzen entsprechend einer vorbestimmten Bewegungsfunktion. Bei dieser Bewegungsfunktion handelt es sich bevorzugt um eine zyklische Hin- und Herbewegung der jeweiligen Quetschwalze 1 in deren axialer Richtung entsprechend einer Sägezahn- oder Sinusfunktion oder einer vergleichbaren bevorzugt zeitlich stetig ausgeführten Bewegungsfunktion. Diese wird dabei in diskreten Schritten gleicher Schrittgröße ausgeführt.

Der präzise Aufbau der Anlage ermöglicht die mikrometergenaue Zustellung der beweglichen Quetschwalze gegen die feststehende Quetschwalze. Störungen im Bereich des Walzenspalts werden einerseits durch die Abdeckungen 46 und 47 reduziert und können andererseits mittels der Steuerung oder Regelung 88 minimiert werden, wie vorstehend anhand der Fig. 1 beschrieben. Insbesondere können die Temperatureinflüsse im Bereich des Walzenspalts messtechnisch über ein Pyrometer erfasst werden und der Walzvorgang entweder manuell oder automatisch ausgeregelt werden. Durch den Aufbau der Anlage ist die mikrometergenaue Manipulation des Innendurchmessers eines gequetschten Rohres in einer bisher nicht gekannten Genauigkeit (gemessen wurden beispielsweise ± 20 µm) möglich. Die Quetschwalzen können in Mikrograden lagegeregelt werden, sodass der jeweils geringste Spaltbreitenfehler der beiden Quetschwalzen ermittelt werden kann. Die gleichlaufgeregelten Rotationsachsen der Quetschwalzen können steuerungstechnisch mit einer Differenzgeschwindigkeit gefahren werden, um Krümmungseffekten, bedingt durch die Umlenkung des Glasstranges, vorzubeugen. Die Abzugsgeschwindigkeit wird durch die Ziehmaschine vorgegeben, die Walzgeschwindigkeit kann mit einer davon abweichenden Geschwindigkeit betrieben werden, um entweder einen Durchhang zu erzeugen oder einen Zug auf den Glasstrang auszuüben, welcher sich maßgeblich auf die Geometrie des gequetschten Rohres auswirkt. Der Steuer- und Regelkreis der Anlage wird aus einem optischen Messsystem, dass der Innendurchmesser des gequetschten Rohres mikrometergenau vermisst und den Präzisionsstellmotoren der jeweiligen Achsen (Rotationsachsen der Walzen, Zustellachsen) gebildet. Weiterhin wird das Walzenpaar über einen weiteren Stellmotor schrittweise verfahren, um einen vorzeitigen Verschleiß der Walzen, bedingt durch den hohen Temperatureinfluss des noch plastischen Glases zu minimieren.

Die Anlage und speziell die Quetschwalzen können luft- bzw. wassergekühlt werden, um die Temperatureinflüsse durch die am Einsatzort gegebenen Bedingungen zu minimieren.

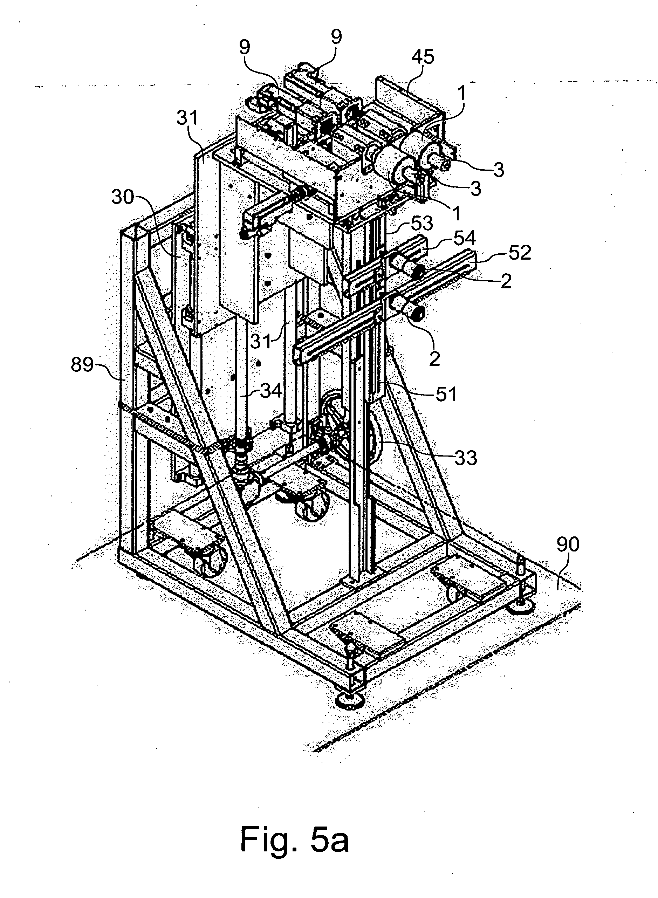

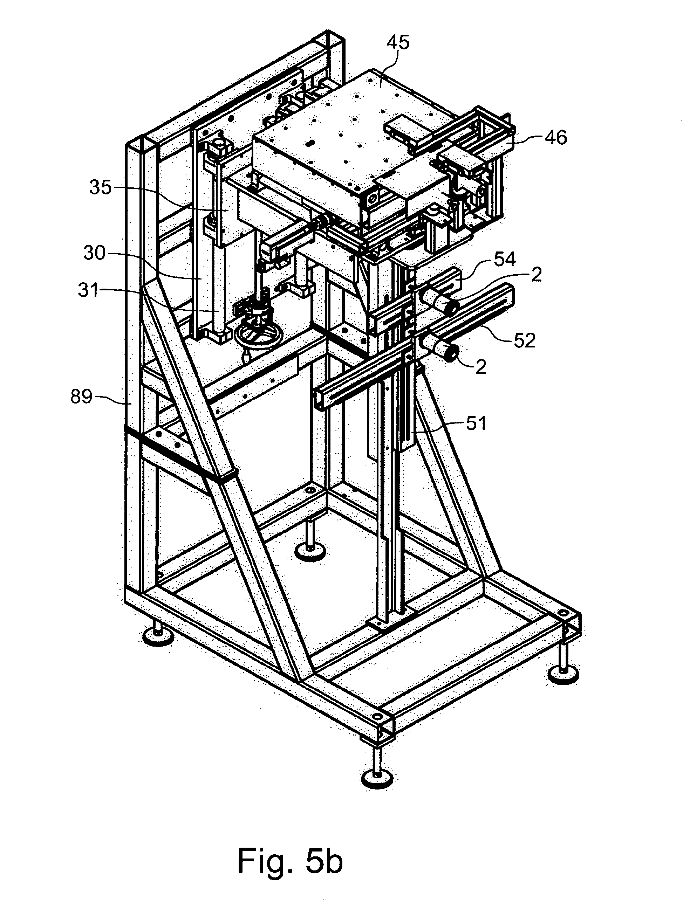

Die Figuren 5a und 5b zeigen zwei weitere Perspektivansichten einer Vorrichtung zur Umformung gemäß der vorliegenden Erfindung.

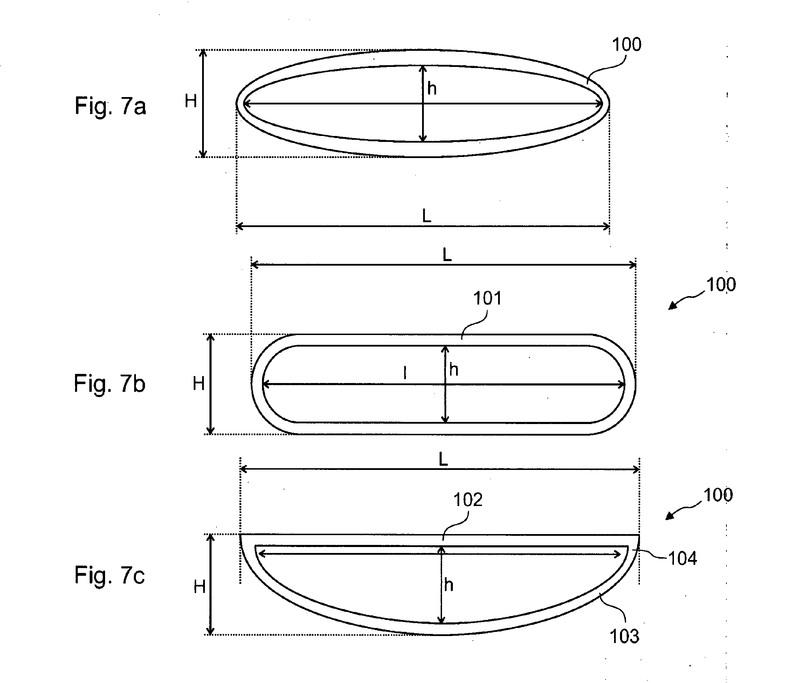

Die Figuren 7a bis 7e zeigen verschiedene Beispiele für Glasrohre mit nicht-kreisrundem Querschnitt, die nach der vorliegenden Erfindung mit hoher Präzision hergestellt werden können.

Gemäß der Fig. 7a ist ein Ovalrohr 100 ausgebildet, dessen Höhe H kleiner ist als die maximale Querabmessung L. Die lichte Weite in Richtung der kleinen Halbachse ist mit h bezeichnet. Die Wandstärke eines solchen Ovalrohrs kann über den gesamten Umfang konstant sein oder, so wie in der Fig. 7a dargestellt, stetig und spiegelsymmetrisch variieren. Ein solches Ovalrohr kann beispielsweise als Vorsatzfilter für LEDs von Flachbildschirmen verwendet werden. Eine um 30% höhere Lichtausbeute konnte festgestellt werden. Ein solches Ovalrohr kann gemäß weiteren bevorzugten Verwendung nach der vorliegenden Erfindung auch als Mediumrohr für Photobioreaktoren zur Bestrahlung von Mikroorganismen, die in dem Mediumrohr aufgenommen sind, oder für solarthermische Anlagen zur Erwärmung eines Fluids, welches das Mediumrohr durchströmt, mittels Sonnenlicht verwendet werden, wie nachfolgend ausgeführt.

Gemäß der Fig. 7b ist das umgeformte Rohr 100 im Wesentlichen rechteckförmig, mit halbkreisförmig gerundeten Seitenkanten und zwei zueinander parallel verlaufenden Längsseiten 101 mit konstanter Wandstärke.

Gemäß der Fig. 7c weist das Glasrohr 100 eine plane Längsseite 102 sowie eine spiegelsymmetrisch und konvex gekrümmte Fläche 103 auf, wobei die Biegeradien in den beiden Eckbereichen 104 sehr klein sind.

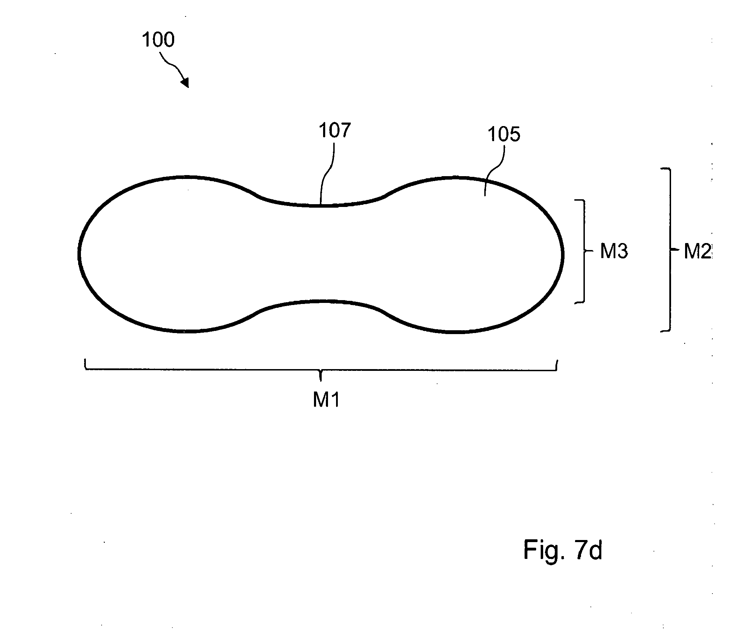

Gemäß der Fig. 7d ist das umgeformte Glasrohr 100 insgesamt hantelförmig ausgebildet, mit zwei symmetrisch ausgebildeten ovalen Seitenflügeln 105 einer Höhe M2, die über einen schmäler ausgebildeten Verbindungssteg 107 einer Höhe M3 miteinander verbunden sind. Die Erfinder haben beobachtet, dass sich ein solches Glasrohr bei üblichen Prozessparametern für die Ausbildung eines Ovalrohrs, wie vorstehend anhand der Fig. 7a beschrieben, ausbildet, wenn die mittleren Seitenwandabschnitte auf der Breitseite des Glasrohrs nach der Umformung im noch heißen, plastisch verformbaren Zustand geringfügig einfallen. Diese mittleren Seitenwandabschnitte können dabei stärker einfallen, als in der Fig. 7d abgebildet, wobei die Seitenwandabschnitte einander in der Mitte des Glasrohrs nicht berühren, um dem hantelförmigen Rohrprofil eine vergleichsweise hohe Stabilität zu verleihen.

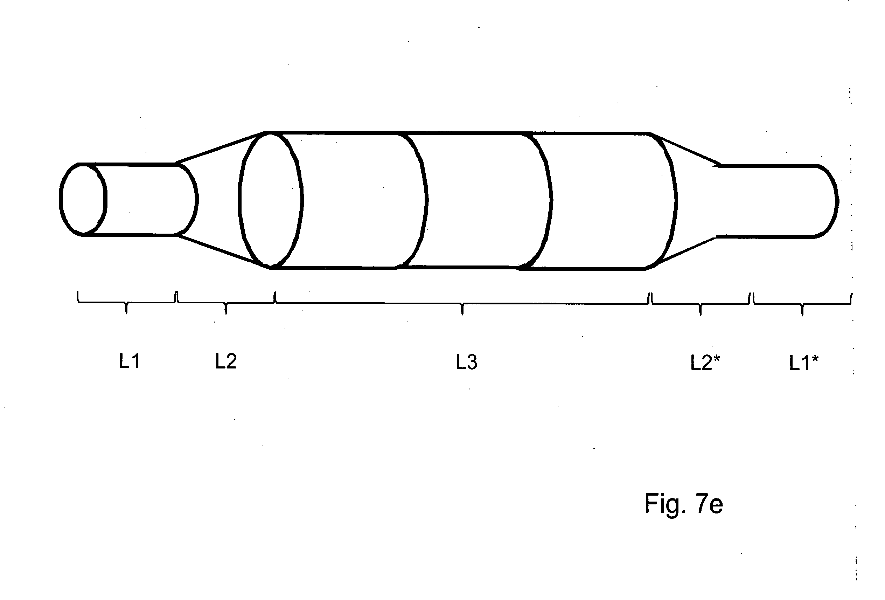

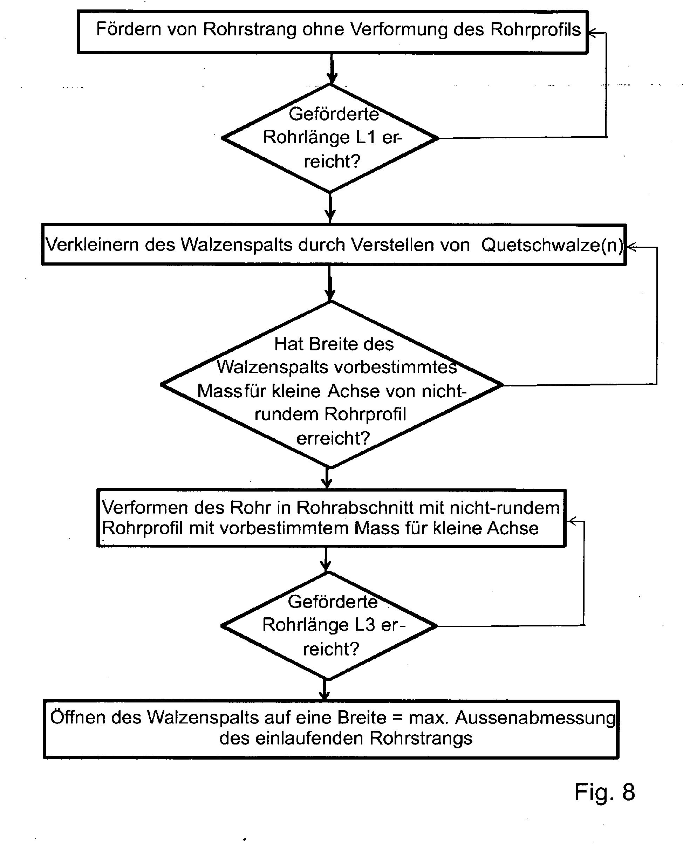

Im Sinne der vorliegenden Erfindung weisen Glasrohre, wie sie vorstehend anhand der Figuren 7a bis 7d beschrieben wurden, Endabschnitte mit einem kreisförmigen Profil auf, wie nachfolgend anhand der Figuren 7e und 8 beschrieben.

Die Fig. 7e zeigt den allgemeinen Aufbau eines Glasrohrs, das nach einem Verfahren gemäß der vorliegenden Erfindung hergestellt ist. Dieses weist einen zentralen Abschnitt L3 mit einem von der Kreisform abweichenden Profil auf, beispielsweise ausgebildet als Ovalrohr, wie vorstehend anhand der Fig. 7a beschrieben. Das Glasrohr weist ferner zwei offene, d.h. nicht abgeschmolzene oder verschlossene, Endabschnitte L1 und L1* auf, die ein kreisförmiges Profil aufweisen, das bevorzugt dem kreisförmigen Profil des in den Walzenspalt zwischen den Quetschwalzen einlaufenden Glasrohrs entspricht, weil das Glasrohr in den Endabschnitten L1 und L1* nicht umgeformt wurde. Die Endabschnitte L1 und L1* gehen über Übergangsabschnitte L2 und L2* stetig in den zentralen Abschnitt L3 mit dem von der Kreisform abweichenden Profil über.

Der zentrale Abschnitt L3 wird durch Umformen des in den Walzenspalt zwischen den Quetschwalzen einlaufenden Glasrohrs ausgebildet. In diesem zentralen Abschnitt L3 entspricht die Breite der Breitseite des Glasrohrs dem Abstand der den Walzenspalt ausbildenden Quetschwalzen. Die Endabschnitte L1 und L1* sind bevorzugt nicht umgeformt. Vielmehr entspricht ihr kreisförmiges Profil dem Ausgangsprofil des in den Walzenspalt zwischen den Quetschwalzen einlaufenden Glasrohrs vor der Umformung des zentralen Abschnitts L3, weil während der Ausbildung dieser Endabschnitte L1 und L1* die Spaltbreite des Walzenspalts größer oder gleich der Außenabmessung des in den Walzenspalt einlaufenden Ausgangsprofils ist. Der Beginn der Übergangsabschnitte L2, L2* zu den Endabschnitten L1, L1* markiert diejenige Spaltbreite des Walzenspalts zwischen den Quetschwalzen, die der Außenabmessung des in den Walzenspalt einlaufenden Ausgangsprofils entspricht. Beim weiteren Verkleinern des Walzenspalts zwischen den Quetschwalzen wird das Ausgangsprofil zunehmend umgeformt, was sich in einer stetigen Formgebung der Übergangsabschnitte L2, L2* ausdrückt. Das Ende der Übergangsabschnitte L2, L2* zu dem zentralen Abschnitt L3 markiert diejenige Spaltbreite des Walzenspalts zwischen den Quetschwalzen, die einem maximalen Umformungsgrad des in den Walzenspalt einlaufenden Ausgangsprofils bzw. einer minimalen Außenabmessung des umgeformten Glasrohrs entspricht, also beispielsweise der Höhe h der Breitseite des Ovalrohrs 100 gemäß der Fig. 7a. Bei dem Rohr gemäß der Fig. 7e sind Umfangslänge des zentralen Abschnitts L3, also des von der Kreisform abweichenden Profils, und die Umfangslängen der Endabschnitte L1, L1* des Rohrs mit dem kreisförmigen Profil gleich.

Die Profile der Abschnitte L2, L3 und L1* können dabei durch Vorgabe der Form der Quetschwalzen sowie deren Betriebsweise geeignet vorgegeben und in einfacher Weise variiert werden. So kann beispielsweise der zentrale Abschnitt L3 insgesamt tropfenförmig ausgebildet werden, wenn die Achsen der Quetschwalzen, welche den Walzenspalt ausbilden, während der Umformung des zentralen Abschnitts so verstellt werden, dass diese V-förmig aufeinander zulaufen. Oder das Profil des zentralen Abschnitts L3 kann, in Axialrichtung betrachtet, variieren, wenn die Quetschwalzen, welche den Walzenspalt ausbilden, ein nicht-kreisförmiges Profil aufweisen, insbesondere ein ovalen oder mehreckiges Profil. Oder der zentrale Abschnitt L3 kann mit einem Profil, wie dieses beispielhaft in der Fig. 7d dargestellt ist, ausgebildet werden, wenn die Quetschwalzen, welche den Walzenspalt ausbilden, ein rotationssymmetrisches Profil mit von der Linienform abweichender Kontur aufweisen, insbesondere wenn die Quetschwalzen gemeinsam einen spiegelsymmetrischen Walzenspalt ausbilden und die Kontur der jeweiligen Quetschwalze eine Vertiefung und seitlich dazu und symmetrisch zwei Vorsprünge aufweist. Selbstverständlich können auch kompliziertere Profile mit mehr als nur einer Einbauchung im zentralen Abschnitt des Rohrs ausgebildet werden, wenn die Quetschwalzen, die den Walzenspalt ausbilden, zwei oder mehr als zwei Vertiefungen aufweisen.

Dabei können die Quetschwalzen aktiv angetrieben sein und/oder zusätzlich geheizt werden. Ferner können die Quetschwalzen axial verstellt und/oder deren Anstellwinkel verändert werden.

Die Fig. 8 zeigt ein schematisches Flussdiagramm eines Verfahrens gemäß der vorliegenden Erfindung zur Herstellung des in der Fig. 7e gezeigten Glasrohrs. Zunächst wird ein Rohr, das ein kreisförmiges Ausgangsprofil aufweist, bereitgestellt. Hierzu kann das Material mittels eines geeigneten Herstellungsverfahrens kontinuierlich hergestellt werden, beispielsweise durch Extrudieren eines Kunststoffs, bevorzugt eines transparenten Kunststoffs, oder durch kontinuierliches Ziehen eines Glasrohrstrangs aus einer Glasschmelze, beispielsweise mit Hilfe eines Down-Draw-Verfahrens, Vello-Verfahrens oder Danner-Verfahrens. Anschließend wird das Rohr in einem plastisch verformbaren Zustand durch einen Walzenspalt gefördert, der von zumindest zwei Quetschwalzen ausgebildet wird. Je nach Material des Rohrs kann hierzu eine gewisse Erwärmung des Rohrs erforderlich sein. Oder das Rohr wird unmittelbar nach seiner Herstellung, beispielsweise durch Ziehen eines Glasrohrstrangs aus einer Glasschmelze oder Extrudieren eines Kunststoffrohrs weiter zu dem Walzenspalt gefördert. In diesem Zustand weist der Walzenspalt eine erste Spaltbreite auf, die größer oder gleich einer Außenabmessung des Ausgangsprofils des Rohrs ist, also des Außendurchmessers des in den Walzenspalt einlaufenden Rohrstrangs. In diesem Betriebszustand erfolgt bevorzug keine Umformung des Rohrs in dem Walzenspalt, da die Quetschwalzen nicht an dem Außenumfang des Rohrstrangs anliegen.

Nach dem Ablängen eines zuvor hergestellten Rohrs wird die in den Walzenspalt geförderte Rohrlänge gemessen, beispielsweise mit Hilfe des Längenmesssgeräts 91 (vgl. Fig. 1). Solange die gemessene Rohrlänge einen vorbestimmten Wert L1 noch nicht überschritten hat, verbleiben die Quetschwalzen in ihrem Ruhezustand, ohne dass das Rohr in dem Walzenspalt umgeformt wird. Erreicht die gemessene Rohrlänge den vorbestimmten Wert L1, wird damit begonnen, die Breite des Walzenspalts durch Verstellen von zumindest einer Quetschwalze in einer Richtung senkrecht zu deren axialer Richtung zu verkleinern. Der Zeitpunkt der unmittelbaren Anlage der Quetschwalzen am Außenumfang des in den Walzenspalt einlaufenden Rohrstrangs markiert dabei den Beginn des Übergangsbereichs L2. Durch weiteres Verkleinern der Breite des Walzenspalts wird das Rohr zunehmend umgeformt, bis schließlich die Breite des Walzenspalts ein vorbestimmtes Maß (zweite Spaltbreite) erreicht hat, das beispielsweise der kleinen Achse des herzustellenden Ovalrohrs entspricht. Dieser Zeitpunkt markiert das Ende des Übergangsabschnitts L2 und den Beginn des zentralen Abschnitts L3 des Glasrohrs mit dem von der Kreisform abweichenden Profil.

Nach Erreichen der zweiten Spaltbreite, die kleiner ist als die Außenabmessung des Ausgangsprofils des in den Walzenspalt einlaufenden Rohrstrangs, wird das Rohr in dem plastisch verformbaren Zustand zu dem von der Kreisform abweichenden Profil L3 umgeformt. Denkbar ist hier die Umformung zu einem Ovalrohr, wie beispielhaft in der Fig. 7a dargestellt, aber auch zu beliebigen anderen Profilen, die von der idealen Kreisform abweichen und beispielhaft vorstehend anhand der Figuren 7b bis 7d beschrieben wurden. In diesem Betriebszustand wird die geförderte Rohrlänge auch weiterhin gemessen. Solange die gemessene Rohrlänge einen vorbestimmten Wert L3 noch nicht überschritten hat, verbleiben die Quetschwalzen in dem Zustand mit der zweiten Spaltbreite, in welchem das Rohr in dem Walzenspalt umgeformt wird. Erreicht die gemessene Rohrlänge den vorbestimmten Wert L3, wird damit begonnen, die Breite des Walzenspalts durch Verstellen von zumindest einer Quetschwalze in einer Richtung senkrecht zu deren axialer Richtung erneut zu vergrößern. Der Zeitpunkt des Beginns dieser Vergrößerung des Walzenspalts markiert dabei den Beginn des hinteren Übergangsbereichs L2*. Durch weiteres Vergrößern der Breite des Walzenspalts wird das Rohr in zunehmend geringerem Maße umgeformt, bis schließlich die Breite des Walzenspalts erneut größer oder gleich der Außenabmessung des Ausgangsprofils ist (dritte Spaltbreite, die gleich der zweiten Spaltbreite sein kann). Dieser Zeitpunkt markiert das Ende des hinteren Übergangsabschnitts L2* und den Beginn des hinteren Endabschnitts L1*, in dem das Rohr erneut ein kreisförmiges Profil aufweist, das insbesondere dem Profil des nicht umgeformten und in den Walzenspalt einlaufenden Ausgangsprofils entspricht. Abschließend wird das Rohr an geeigneter Stelle in dem hinteren Endabschnitt L1* abgelängt.

Wie dem Fachmann ohne weiteres ersichtlich sein wird, können auf diese Weise Rohre hergestellt werden, deren Endabschnitte L1, L* kreisförmig sind, also mittels herkömmlicher Rohrverbindungstechnologien mit Anschlussmitteln (beispielsweise Zulauf und Ablauf) oder benachbarten Rohren verbunden werden können, die jedoch zumindest einen zentralen Abschnitt L3 mit einem geeignet von der Kreisform abweichenden Profil aufweisen. Auf diese Weise können erfindungsgemäß Rohre mit einem abschnittsweise von der Kreisform abweichenden Profil dennoch mittels bewährter Rohrverbindungstechnologien mit Anschlussmitteln oder benachbarten Rohren verbunden werden. Nach dem erfindungsgemäßen Verfahren, wie vorstehend anhand der Fig. 8 beispielhaft beschrieben, können auch in axialer Richtung des Rohrs beliebige Profilverläufe eingestellt werden. Grundsätzlich werden dabei symmetrische Profilverläufe bevorzugt, wie in der Fig. 7e dargestellt, da diese in der Regel spannungsärmer hergestellt werden können. Grundsätzlich denkbar sind jedoch auch beliebige andere in axialer Richtung des Rohrs asymmetrische Profilverläufe.

Anwendungsgebiete solcher Rohre, insbesondere Glasrohre, sind beispielsweise: Ovalrohre mit hoher geometrischer Genauigkeit für die Verwendung von hermetischen Verpackungen für Halbleiter-Nanopartikel, bei denen eine hohe geometrische Genauigkeit (Spaltmaß) wichtig ist; Kolben- bzw. Hüllrohre für Entladungslampen, insbesondere Blitzlampen bei denen das Einbaumaß, jedoch nicht die Lichtausbeute minimiert werden soll.

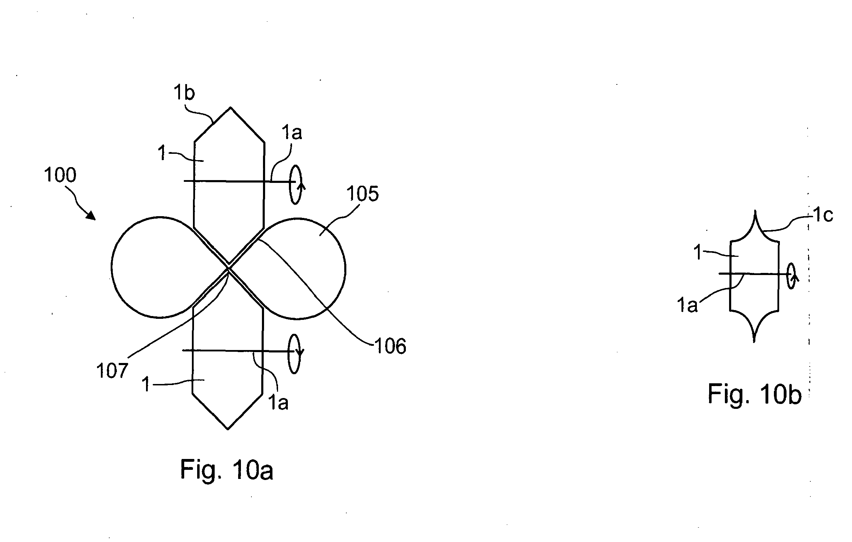

Anhand der Figuren 10a und 10b wird nachfolgend ein Verfahren gemäß einer weiteren Ausführungsform der vorliegenden Erfindung beschrieben. In der Fig. 10a ist in einer schematischen Schnittansicht der Bereich des Walzenspalts dargestellt, der von zwei Quetschwalzen 1 ausgebildet wird. Das Glasrohr 100 durchläuft den Walzenspalt senkrecht zur Zeichenebene. Die Quetschwalzen 1 drehen sich um die jeweilige Achse 1a. Abweichend zur Ausführungsform gemäß den Figuren 2 bis 6 weisen die Quetschwalzen 1 auf Ihrem Außenumfang einen Vorsprung 1b auf, der bei dem dargestellten Ausführungsbeispiel die Form eines gleichschenkligen Dreiecks auf, wenngleich die vorliegende Erfindung grundsätzlich nicht auf dieses Profil beschränkt sein soll. In der Stellung gemäß der Fig. 10a bilden die Quetschwalzen 1 einen Walzenspalt aus, der sich ausgehend von der schmälsten Stelle, nämlich dort, wo die Scheitel der Vorsprünge 1b einander gegenüber liegen, symmetrisch und keilförmig verbreitert. Durch diesen Walzenspalt wird das Glasrohr 100 so stark verformt, dass es im zentralen Bereich zu einer Einschnürung 107 kommt, wo sich die Oberseite und Unterseite des Glasrohrs berühren und unter Ausbildung eines Verbindungsstegs 107 miteinander verschmelzen. Ausgehend von dem Verbindungssteg 107 erstrecken sich schräge Verbindungsschenkel 106, deren Form und Neigung durch das Profil der Vorsprünge 1b der Quetschwalzen 1 vorgegeben wird, die schließlich in zwei kugelförmige Seitenflügel 105 übergehen. Ein solchermaßen ausgebildetes Glasrohr 100 gibt somit zwei Strömungskanäle vor, die von den Seitenflügeln 105 ausgebildet werden. Im Bereich des Verbindungsstegs 107 sind diese Strömungskanäle voneinander getrennt, sodass beispielsweise in diesem Bereich Fluide in entgegen gesetzte Richtungen strömen können.

Die Fig. 10b zeigt ein weiteres Ausführungsbeispiel für eine Quetschwalze 1, die einen Vorsprung 1c mit einer konkaven Wölbung aufweist. Dies führt entsprechend zur Fig. 10a im Bereich des Verbindungsschenkels zu einer entsprechend konvexen Wölbung des Glasrohrs (nicht dargestellt).

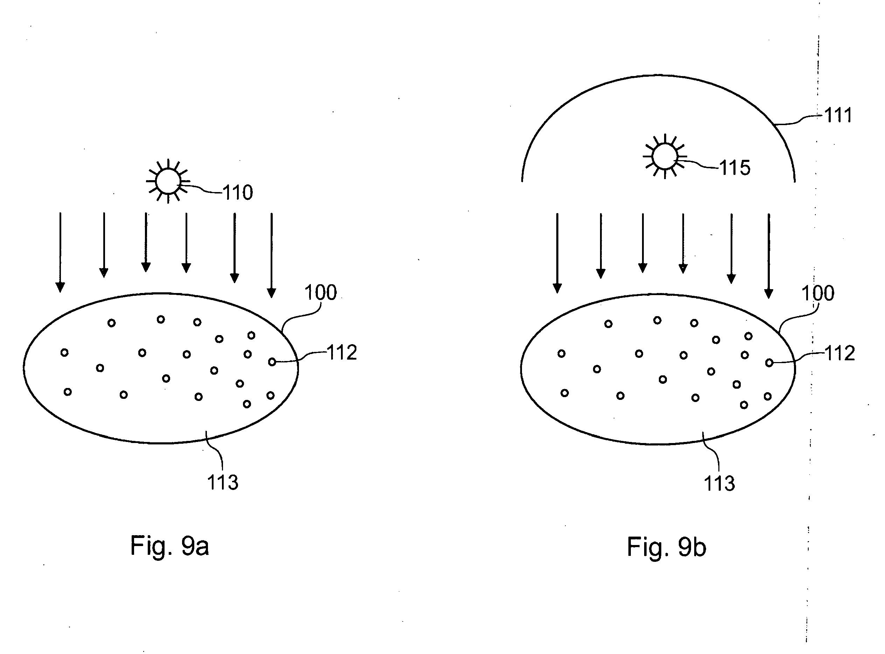

Anhand der Figuren 9a bis 9c werden nachfolgend zwei bevorzugte Anwendungen eines Glasrohrs beschrieben, das nach einem Verfahren gemäß der vorliegenden Erfindung hergestellt ist. Die Fig. 9a zeigt die Verwendung eines solchen transparenten Rohrs in einem Photobioreaktor zum Züchten von Mikroorganismen durch Bestrahlung mit natürlichem Licht. In dem transparenten Rohr 100, das aus Glas oder einem geeigneten transparenten Kunststoff ausgebildet sein kann, befindet sich eine Flüssigkeit 113, welche die zu züchtenden Mikroorganismen 112, beispielsweise Algen, als Matrix aufnimmt. Das Rohr 100 ist über seine Endabschnitte (nicht dargestellt), die das kreisförmige Profil aufweisen, mit Anschlussmitteln, also einem Zu- und Ablauf (nicht dargestellt), oder mit weiteren Rohren des gleichen Typs verbunden, ggf. über Zwischen- oder Verbindungsstücke, sodass die Flüssigkeit 113 mit den Mikroorganismen 112 kontinuierlich oder getaktet durch den Photobioreaktor gepumpt werden kann. Das Rohr weist zumindest einen zentralen Abschnitt mit einem von der Kreisform abweichenden Profil auf und ist im Übrigen so ausgebildet, wie vorstehend anhand der Fig. 7e beschrieben. Dieser zentrale Abschnitt ist beispielsweise als das dargestellte Ovalrohr ausgebildet. Bei der Verwendung eines solchen Rohrs ist der zentrale Abschnitt (L3) des Rohrs 100, also beispielsweise das dargestellte Ovalrohr, mit seiner breiteren Seite der natürlichen Lichtquelle (Sonne) 110 zugewandt angeordnet. Genauer gesagt ist die Hauptachse, welche durch die beiden Brennpunkte des ovalen Rohrs 100 verläuft, senkrecht zur Lichteinfallrichtung bzw. zu einer über den üblichen Bestrahlungsverlauf gemittelten Lichteinfallrichtung ausgerichtet.

Wie dem Fachmann ohne Weiteres ersichtlich sein wird, kann man in der Anordnung nach der Fig. 9a die natürliche Lichtquelle auch mittels Hohlspiegeln oder ähnlichen optischen Abbildungseinrichtungen auf das Rohr 100 geeignet abbilden, wobei in der Fig. 9a dann die dargestellten Pfeile die Richtung des Sonneneinfalls symbolisieren sollen. In diesem Fall wäre dann der zentrale Abschnitt (L3) des Rohrs, also beispielsweise des dargestellten Ovalrohrs, in entsprechender Weise mit seiner breiteren Seite dem Sonnenlicht bzw. der Richtung des Sonneneinfalls zugewandt angeordnet. Insbesondere für den Fall, dass die Sonnenstrahlung ohne optische Abbildungseinrichtungen auf das Rohr 100 einfällt, wird das Rohr 100 im Allgemeinen nicht exakt (im mathematischen Sinne) mit seiner Breitseite der Richtung des Sonneneinfalls zugewandt sein, sondern nur ungefähr, da das Rohr 100 im Allgemeinen nicht dem sich ändernden Sonnenstand entsprechend nachgeführt wird. Natürlich könnte eine solche Nachstellung der Orientierung der Rohre 100 des Photobioreaktors grundsätzlich auch vorgesehen werden, um eine optimale Einstrahlung des Sonnenlichts in das erfindungsgemäße Rohr 100 zu ermöglichen. Alternativ wird die Abbildung des Sonnenlichts auf das Rohr 100 durch geeignetes Verstellen des Hohlspiegels oder von ähnlichen optischen Abbildungseinrichtungen dem Sonnenstand entsprechend nachgestellt.

Die Fig. 9b zeigt die Verwendung eines transparenten Rohrs 100, das nach einem Verfahren gemäß der vorliegenden Erfindung hergestellt ist, in einem Photobioreaktor zum Züchten von Mikroorganismen durch Bestrahlung mit künstlichem Licht. Gemäß der Fig. 9b ist die künstliche Lichtquelle 115 im Brennpunkt eines Hohlspiegels 111 angeordnet ist, der sich entlang dem jeweiligen zentralen Abschnitt und parallel zu diesem erstreckt, sodass das Licht der künstlichen Lichtquelle 110 in einen aufgeweiteten Lichtstrahl abgebildet wird, der das Rohr 100 bestrahlt. Dabei ist der zentrale Abschnitt (L3) des Rohrs 100, also beispielsweise das dargestellte Ovalrohr, mit seiner breiteren Seite der Lichtquelle 115 zugewandt angeordnet. Genauer gesagt ist die Hauptachse, welche durch die beiden Brennpunkte des ovalen Rohrs 100 läuft, senkrecht zur Lichteinfallrichtung ausgerichtet, wie diese durch den Hohlspiegel 111 und die Anordnung der Lichtquelle 115 vorgegeben wird.

Zahlenbeispiel: Im Vergleich zu einem runden Rohr hat ein ovales Rohr (bei gleichem Umfang) und einem Hauptachsenverhältnis von beispielsweise 1,42:1 eine um den Faktor 1,16 vergrößerte Fläche. Aufgrund des etwas geringeren Querschnitts fließen die Algen aber schneller im ovalen Bereich als im runden Bereich und werden um den Faktor 1,06 kürzer belichtet. Zusammengenommen bleibt erfindungsgemäß jedoch ein positiver Effekt mit einem Faktor 1,09. Dieser Faktor entspricht einem gegenüber einem vollständig runden Rohr erhöhten Algenwachstum. In Photobioreaktoren können Algen so in Rohren gezüchtet, in denen mehrere Rohre zu quasi-endlos Rohren verbunden sind (Länge beispielsweise bis zu 50m) und vertikal übereinander mit Ständern angeordnet und der Sonne oder eine künstlichen Lichtquelle ausgesetzt sind. Die Kombination von ovalen Rohren mit runden Enden, wie vorstehend beschrieben, ist vorteilhaft, um die Vorteile der erhöhten Lichtausbeute und der herkömmlicher Verbindungstechniken von runden Rohren zu nutzen.

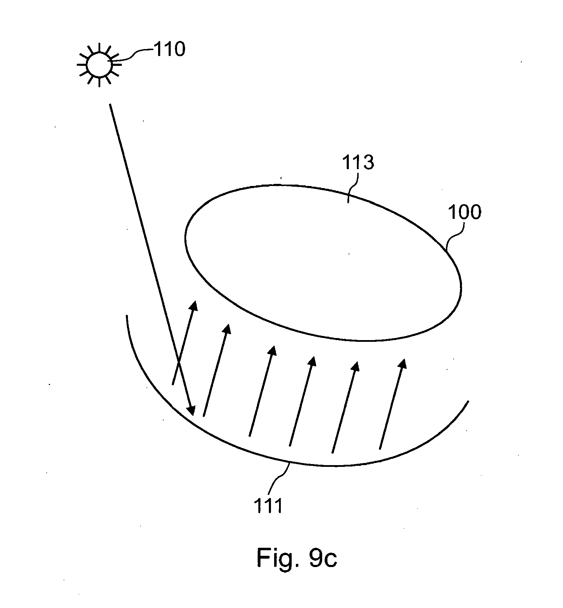

Die Fig. 9c zeigt eine weitere Verwendung eines Rohrs 100, das nach einem Verfahren gemäß der vorliegenden Erfindung hergestellt ist, für Anwendungen in der Solarthermie zur Erwärmung eines Fluids mittels Sonnenlicht. In dem transparenten Rohr 100, das aus Glas oder einem geeigneten Kunststoff ausgebildet sein kann, befindet sich eine Flüssigkeit 113, die erwärmt werden soll. Das Rohr 100 ist über seine Endabschnitte (nicht dargestellt), die das kreisförmige Profil aufweisen, mit Anschlussmitteln, also einem Zu- und Ablauf (nicht dargestellt), oder mit weiteren Rohren des gleichen Typs verbunden, sodass die Flüssigkeit 113 kontinuierlich oder getaktet durch die solarthermische Anlage gepumpt werden kann. Das Rohr weist zumindest einen zentralen Abschnitt mit einem von der Kreisform abweichenden Profil auf und ist im Übrigen so ausgebildet, wie vorstehend anhand der Fig. 7e beschrieben. Dieser zentrale Abschnitt ist beispielsweise als das dargestellte Ovalrohr 100 ausgebildet. Bei der Verwendung eines solchen Rohrs ist der zentrale Abschnitt (L3) des Rohrs, also beispielsweise das dargestellte Ovalrohr, mit seiner breiteren Seite einer Lichtquelle zugewandt angeordnet. Gemäß der Fig. 9c wird dabei das Licht der Sonne 110 über einen Hohlspiegel 111 auf das Rohr 100 abgebildet, das sich entlang dem jeweiligen zentralen Abschnitt und parallel zu diesem erstreckt, sodass das Sonnenlicht der Lichtquelle in einen aufgeweiteten Lichtstrahl abgebildet wird, der das Rohr 100 bestrahlt. Bei dieser Geometrie ist das Rohr 100 mit seiner breiteren Seite dem Hohlspiegel 111 zugewandt angeordnet, der hier als Lichtquelle zur Erwärmung des Fluids 113 in dem Rohr 100 dient. Üblicherweise wird der Hohlspiegel 111 entsprechend dem sich verändernden Sonnenstand nachgeführt werden. Bei einfacheren, weniger effizienten Solarsystemen braucht dies jedoch nicht unbedingt der Fall zu sein, in welchem Fall die vorstehend beschriebene Orientierung des Rohrs nicht unbedingt exakt (im mathematischen Sinne) sein wird, sondern vielmehr einer optimalen Orientierung allenfalls bestmöglich angenähert sein wird. Durch die ovalen Rohrabschnitte können Toleranzen in der Spiegelgeometrie und im Nachführungssystem zur Sonne größer sein und allgemein Kosten eingespart werden.

Als Materialien für das Rohr, das nach einem Verfahren gemäß der vorliegenden Erfindung hergestellt ist, können beliebige verformbare Materialien verwendet werden, beispielsweise Glas, Kunststoff oder Kunststoff-Verbundmaterialien. Für die bevorzugten Anwendungen, bei denen ein in dem Rohr aufgenommenes Fluid bestrahlt werden soll, ist das Material des Rohrs in dem Wellenlängenbereich des bestrahlenden Lichts geeignet transparent. Bevorzugt werden hierzu transparente Glassorten, beispielsweise Duran (Si0₂-81%, B₂0₃ - 13%, N₂0+K₂0-4%, Al₂0₃ - 2%), Kalknatronglas oder Fiolax^{®}, oder transparente Kunststoffe ausgewählt aus einer Gruppe, die umfasst: Polymethylmethacrylat (PMMA), Polycarbonat (PC), Polyamid (PA), Polyethylen (PE) Polypropylen (PP), Polystyrol (PS), Poly-4-methylpenten-1 (PMP), Polyvinylchlorid (PVC), Cycloolefincopolymere (COC), Styrol/Butadien/Styrol-Bolckcopolymer (SBS), Methylmethacrylat/Acrylnitril/Polybutadien/Styrolpfropfcopoylmer (MABS), Aromatische Polyester (APE), Polyestercarbonate (PEC), Cellulosepropionat (CP), Polyetherfluorethylen (PTFE), Poylethersulfon (PES).

Die Rohrlänge können 0,20m - 10m betragen, der Durchmesser im runden Bereich kann größer als 8 mm und kleiner als 200 mm beträgen, die Wandstärken können im Bereich 0,5 bis 10mm liegen. Ovale Rohre im Sinne der vorliegenden Anmeldung können mit Haupachsen a und b längster bzw. kürzester Durchmesser) und Gröenachsenverhältnisen 1 < a/b < 2 und insbesondere 1,2 < a/b < 1,6 bereitgestellt werden.

Bezugnehmend auf die Fig. 1 kann das Glasrohr entsprechend der stromabwärts des Walzenspalts mittels der Messgeräte 86, 87 erfassten Außenabmessung und/oder Innenabmessung markiert werden, beispielsweise durch Beschriften oder mittels Lasermarkieren. Weiterhin kann das Glasrohr entsprechend der stromabwärts des Walzenspalts mittels der Messgeräte 86, 87 erfassten Außenabmessung und/oder Innenabmessung auch sortiert werden.

Für eine Vorrichtung zum Quetschen von zähplastischem Glasrohr mit einer Innendurchmessertoleranz von +/- 20 µm liegen die Anforderungen an die Toleranzen der Komponenten im Allgemeinen bei einer 10-fachen Genauigkeit der Toleranzvorgabe. Um die Kosten noch in einem erträglichen Rahmen zu halten, wurden die Einzelkomponenten der Vorrichtung auf max. +/-3 µm toleriert. Zu beachten ist der Temperatureinfluss, da eine Temperaturänderung von 1K zu einer Spaltmaßänderung von 1 µm führt. Mittels eines Kühlsystems kann deshalb optional die Temperatur der Anlage möglichst konstant gehalten werden. Die Walzenoberflächen heizen sich aber durch den Glasstrang üblicherweise unkontrolliert auf, was durch die vorstehend beschriebene permanente axiale Verstellung von zumindest einer der Quetschwalzen und bevorzugt von beiden den Walzenspalt ausbildenden Quetschwalzen vermieden werden kann. Dies kann durch eine permanente Überwachung und Steuerung oder Regelung der Spaltbreite des Walzenspalts weiter unterstützt werden.

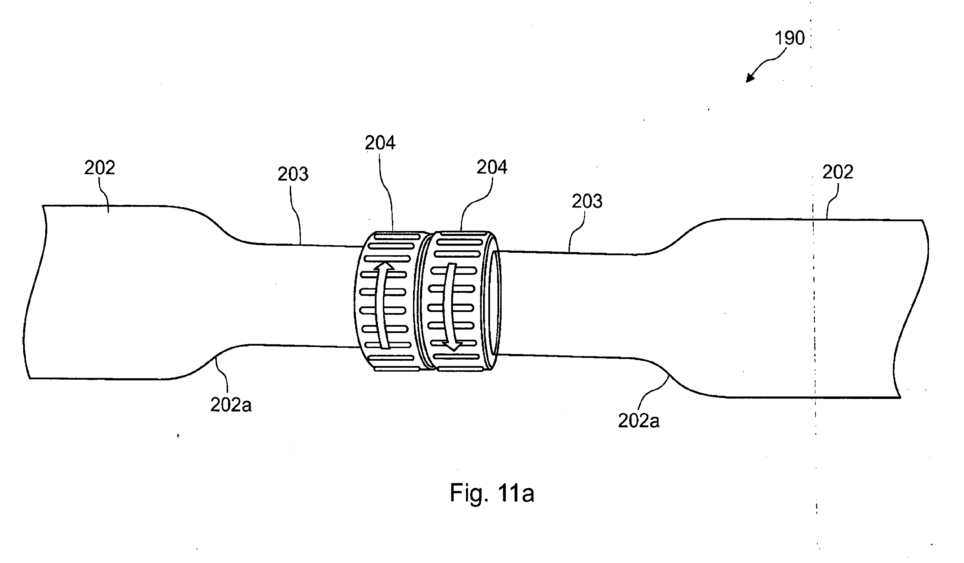

Die Fig. 11a zeigt eine beispielhafte Verbindungsanordnung 190 von zwei Rohren, die nach einem Verfahren gemäß der vorliegenden Erfindung hergestellt sind. Von den Rohren sind nur die Endabschnitte 203 mit kreisförmigem Profil dargestellt, die über die Übergangsbereiche 202a in die jeweiligen Abschnitt 202 mit nicht-kreisförmigem Profil übergehen, wie vorstehend beschrieben. Die beiden Endabschnitte 203 mit kreisförmigem Profil sind jeweils von einem hülsenförmigen Rohrverbinder 204 umgeben. Die beiden Rohrverbinder 204 sind bevorzugt miteinander verbunden, können aber auch einstückig ausgebildet sein. Durch Verdrehen der jeweiligen Rohrverbinder 204, wie in der Fig. 11a durch den Drehpfeil angedeutet, wird eine dichte Verbindung zwischen dem jeweiligen Rohrverbinder 204 und dem jeweiligen Endabschnitt 203 mit kreisförmigem Profil bewirkt. Beispielsweise wird durch Verdrehen des Rohrverbinders eine Endkappe desselben axial verstellt, wodurch ein O-Ring komprimiert wird, welcher den Endabschnitt 203 mit kreisförmigem Profil abdichtet.

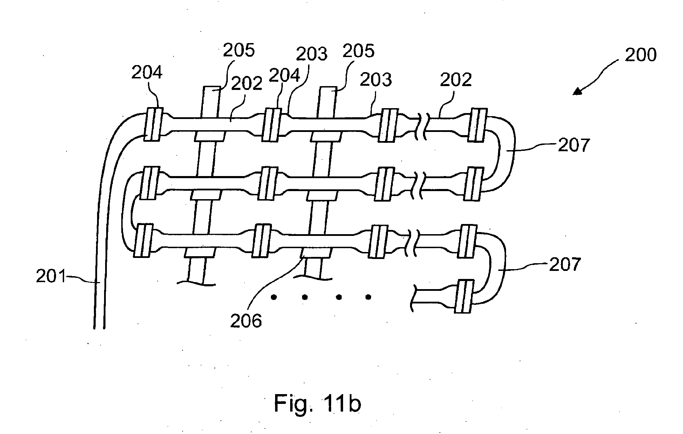

Fig. 11b zeigt in einer schematischen Darstellung einen Photobioreaktor 200 zum Züchten von Mikroorganismen durch Bestrahlung, beispielsweise von Algen, wobei der Photobioreaktor 200 Rohre umfasst, die nach einem Verfahren gemäß der vorliegenden Erfindung hergestellt sind. Wachsenden Algen nutzen Licht und bestimmte Nährstoffe, vor allem Kohlendioxid, lösliche Stickstoffverbindungen und Phosphat. Tageslicht wird normalerweise als Licht verwendet, aber künstliches Licht kann als Alternative zu Tageslicht oder zusätzlich dazu verwendet werden. Eine kleine Menge der Mikroorganismen sind einer Flüssigkeit zugemischt, beispielsweise Wasser, insbesondere Frischwasser oder Meerwasser, oder einer geeigneten Nährflüssigkeit. Der Photobioreaktor 200 umfasst eine Mehrzahl von Rohren, die geeignet transparent sind, sodass durch diese Licht in die Flüssigkeit eindringen kann, die sich in den Rohren befindet. Die Rohre umfassen jeweils Abschnitte 202 mit nicht-kreisförmigem Profil und Abschnitte 204 mit kreisförmigem Profil, wie vorstehend beschrieben. Die Rohre sind über Verbindungselemente 204 miteinander verbunden, die beispielsweise so ausgebildet sind, wie vorstehend anhand der Fig. 11a beschrieben. Eine Mehrzahl von Rohren ist auf diese Weise in Reihe zusammengeschaltet, um eine jeweilige Reihe auszubilden. Die einzelnen Reihen sind über U-Rohre 207 miteinander verbunden, sodass eine insgesamt mäanderförmige Anordnung von Rohren realisiert ist, in welcher die Flüssigkeit mit den Mikroorganismen aufbewahrt ist und geeignet strömt. Die jeweilige erste bzw. letzte Reihe von Rohren kann über eine Verbindungsleitung 201 mit Vorratsbehältern, Pumpen etc. (nicht dargestellt) verbunden sein. Die Rohre sind mittels Haltelementen 206 in einem Gestell 205 gehalten, das eine geeignete Ausrichtung der Rohre ermöglicht, wie vorstehend beschrieben.

Wenngleich vorstehend beschrieben wurde, dass die Rohre an beiden Enden einen Abschnitt mit kreisförmigem Profil aufweisen, wird dem Fachmann ersichtlich sein, dass die Rohre bei oder nach Ihrer Herstellung auch an beliebigen anderen Abschnitten geeignet abgelängt werden können. Denkbar sind beispielsweise Rohre, die einen Abschnitt mit einem kreisförmigem Profil zur Verbindung mit benachbarten Rohren sowie zumindest einen weiteren Abschnitt mit nicht-kreisförmigem Profil aufweisen, wobei die Rohre in diesem Abschnitt mit nicht-kreisförmigem Profil abgelängt werden. Solche Rohre weisen also beispielsweise ein Ende mit einem kreisförmigen Profil und ein entgegen gesetztes ende mit einem nicht-kreisförmigen Profil auf. Nach der Ausführungsform nach der Fig. 10a kann das Rohr beispielsweise auch als hantelförmiges Doppelrohr ausgebildet sein, also ohne jeglichen Endabschnitt mit kreisförmigem Profil, wobei die Verbindung mit den von den Seitenflügeln 105 ausgebildeten Strömungskanälen dann in anderer Weise bewerkstelligt wird, beispielsweise mittels Schläuchen.

Wenngleich vorstehend beschrieben wurde, dass die Vorrichtung zur Umformung von Glasrohren eingesetzt wird, kann die Vorrichtung in entsprechender Weise auch zur Umformung von Rohren aus anderen plastisch dauerhaft verformbaren Materialien eingesetzt werden. Bevorzugt hat das Ausgangsglasrohr einen kreisrunden Querschnitt und erfolgt dann die Umformung zu einem davon abweichenden Profil in zumindest einem zentralen Abschnitt. Die Rohrenden sind in den Bereich der Endabschnitte mit konstantem Profil und offen ausgebildet, also nicht abgeschmolzen oder in anderer Weise verschlossen. Wie dem Fachmann beim Studium der vorstehenden Beschreibung ohne weiteres ersichtlich sein wird, wurde die Erfindung vorstehend nur in beispielhafter Weise und unter Bezugnahme auf Ausführungsbeispiele beschrieben und können zahlreiche Modifikationen vorgenommen werden, ohne von dem allgemeinen Lösungsgedanken und dem Schutzbereich der Erfindung, wie dieser durch die beigefügten Patentansprüche festgelegt ist, abzuweichen. Ferner können die vorstehend beschriebenen Merkmale erfindungsgemäß auch in anderer Weise als vorstehend konkret offenbart miteinander kombiniert werden.

### Bezugszeichenliste

- 1: Quetschwalzen
- 1a: Drehachse
- 1b: dreieckförmiger Vorsprung auf Außenumfang der Quetschwalze 1
- 1c: Vorsprung auf Außenumfang der Quetschwalze 1 mit konkaver Wölbung
- 2: Umlenkrollen
- 3: Drehdurchführung
- 4: Spindelwelle
- 5: Lager
- 6: Lagerblock
- 7: Spindellager
- 8: Kupplung
- 9: Servomotor
- 10: Getriebe
- 11: Flansch
- 12: Verschiebetisch
- 13: Spaltmaßverstellung
- 15: Führungsschiene
- 20: Trägerplatte
- 21: Führungsstange
- 22: Lagerblock
- 23: Lagerblock
- 24: Verstellspindel (für axiale Walzenverstellung)
- 25: Motor (für axiale Walzenverstellung)
- 30: Grundplatte
- 31: Führungsstange
- 32: Lagerblock
- 33: Drehrad (Höhenverstellung)
- 34: Verstellspindel
- 35: Verschiebetisch
- 37: Adapter
- 38: Adapter
- 45: Gehäusedeckel mit Kühlung
- 46: Abdeckung
- 47: Öffnung
- 50: Haltesteg
- 51: Höhenverstellung
- 52: Querverstellung
- 53: Höhenverstellung
- 54: Querverstellung
- 80: Glasrohrherstellungsanlage
- 81: Glasrohrstrang
- 82: Ziehmaschine
- 83: Zugrollenpaar
- 84: Zugrollenpaar
- 85: Gestell
- 86: Außendurchmesser-Messgerät
- 87: Innendurchmesser-Messgerät
- 88: Zentrale Steuerung oder Regelung
- 89: Gestell
- 90: Boden
- 91: Längenmessgerät
- 92: Trennvorrichtung
- 100: Glasrohr
- 101: Längsseite
- 102: plane Längsseite
- 103: konvex gekrümmte Seite
- 104: Eckbereich
- 105: Seitenflügel
- 106: schräger Schenkel
- 107: Einschnürung / Verbindungssteg
- 110: Natürliche Lichtquelle (Sonne)
- 111: Hohlspiegel
- 112: Mikroorganismen
- 113: Flüssigkeit
- 115: künstliche Lichtquelle
- 190: Rohverbindungsanordnung
- 200: Photobioreaktor
- 201: Verbindungsleitung
- 202: Abschnitt des Rohrs mit nicht-kreisförmigem Profil
- 202a: Übergangsbereich
- 203: Abschnitt des Rohrs mit kreisförmigem Profil
- 204: Rohrverbinder
- 205: Gestell
- 206: Halteelement
- 207: U-Rohr

## Patentansprüche

1. Verfahren zur Herstellung eines Rohrs mit einem abschnittsweise von einer Kreisform abweichenden Profil durch Umformen, mit den Schritten:
a) Bereitstellen eines Rohrs (81) aus Glas, Kunststoff oder einem Kunststoff-Verbundmaterial, wobei das Rohr ein kreisförmiges Ausgangsprofil (L1) aufweist;
b) Fördern des Rohrs (81) in einem plastisch verformbaren Zustand durch einen Walzenspalt, der von Quetschwalzen (1) ausgebildet wird und eine erste Spaltbreite aufweist, die größer oder gleich einer Außenabmessung des Ausgangsprofils ist;
c) Verstellen der Quetschwalzen (1), um eine zweite Spaltbreite des Walzenspalts einzustellen, die kleiner ist als die Außenabmessung des Ausgangsprofils, und Umformen des Ausgangsprofils in dem plastisch verformbaren Zustand zu dem von der Kreisform abweichenden Profil (L3); und
d) Verstellen der Quetschwalzen (1), um eine dritte Spaltbreite des Walzenspalts einzustellen, die größer oder gleich der Außenabmessung des Ausgangsprofils ist, und Abtrennen des Rohrs in einem Bereich mit kreisförmigem Profil;
sodass Endabschnitte (L1; L1*) des Rohrs jeweils ein kreisförmiges Profil aufweisen.

2. Verfahren nach Anspruch 1, wobei eine geförderte Rohrlänge des Rohrs (81) gemessen wird, wobei auf Grundlage eines Messwerts für die jeweils geförderte Rohrlänge des Rohrs (81) eine axiale Länge des von der Kreisform abweichenden Profils (L3) und/oder eine axiale Länge von Übergangsbereichen (L2; L2*) zwischen den Endabschnitten (L1; L1*) des Rohrs und dem von der Kreisform abweichenden Profil (L3) eingestellt wird.

3. Verfahren nach Anspruch 2, wobei das Verstellen der Quetschwalzen (1) in den Schritten c) und d) entsprechend einer vorbestimmten Bewegungsfunktion erfolgt, um die Übergangsbereiche (L2; L2*) zwischen den Endabschnitten (L1; L1*) des Rohrs und dem von der Kreisform abweichenden Profil (L3) mit Profilen entsprechend der vorbestimmten Bewegungsfunktion auszubilden.

4. Verfahren nach einem der vorhergehenden Ansprüche, wobei das Umformen des Ausgangsprofils zu dem von der Kreisform abweichenden Profil (L3) im Schritt c) in einem heißen plastisch verformbaren Zustand erfolgt, wobei die Lage von zumindest einer der Quetschwalzen (1) permanent so verändert wird, insbesondere so permanent axial verstellt wird, dass eine Kontaktfläche zwischen der jeweiligen Quetschwalze und dem in den Walzenspalt einlaufenden heißen Rohr permanent variiert wird.

5. Verfahren nach Anspruch 4, wobei das permanente axiale Verstellen der jeweiligen Quetschwalze entsprechend einer vorbestimmten Bewegungsfunktion erfolgt, wobei die vorbestimmte Bewegungsfunktion
eine zyklische Hin- und Herbewegung der jeweiligen Quetschwalze in deren axialer Richtung ist und/oder
in diskreten Schritten gleicher Schrittgröße ausgeführt wird.

6. Verfahren nach einem der vorhergehenden Ansprüche, wobei die Achsen der Quetschwalzen (1), welche den Walzenspalt ausbilden, V-förmig aufeinander zulaufen.

7. Verfahren nach einem der vorhergehenden Ansprüche, wobei die Quetschwalzen (1), welche den Walzenspalt ausbilden, ein nicht-kreisförmiges Profil aufweisen, insbesondere ein ovalen oder mehreckiges Profil.

8. Verfahren nach einem der vorhergehenden Ansprüche, wobei die Quetschwalzen (1), welche den Walzenspalt ausbilden, ein rotationssymmetrisches Profil mit von der Linienform abweichender Kontur aufweisen, wobei die Quetschwalzen insbesondere einen spiegelsymmetrischen Walzenspalt ausbilden, und wobei die Kontur der jeweiligen Quetschwalze insbesondere zumindest eine Vertiefung oder zumindest einen Vorsprung aufweist.

9. Verfahren nach einem der vorhergehenden Ansprüche, wobei die Quetschwalzen (1) aktiv angetrieben sind und/oder zusätzlich geheizt werden, und/oder
wobei die Quetschwalzen (1) axial verstellt und/oder deren Anstellwinkel verändert wird.

10. Verfahren nach einem der vorhergehenden Ansprüche, wobei das Rohr (81) mit dem kreisförmigen Ausgangsprofil (L1) aus einem Glas bereitgestellt wird, insbesondere aus einem transparenten Glas.

11. Verfahren nach einem der Ansprüche 1 bis 9, wobei das Rohr (81) mit dem kreisförmigen Ausgangsprofil (L1) aus einem Kunststoff bereitgestellt wird, insbesondere aus einem transparenten Kunststoff, wobei der Kunststoff bevorzugt ausgewählt ist aus einer Gruppe, die umfasst: Polymethylmethacrylat (PMMA), Polycarbonat (PC), Polyamid (PA), Polyethylen (PE) Polypropylen (PP), Polystyrol (PS), Poly-4-methylpenten-1 (PMP), Polyvinylchlorid (PVC), Cycloolefincopolymere (COC), Styrol/Butadien/Styrol-Bolckcopolymer (SBS), Methylmethacrylat/Acrylnitril/Polybutadien/Styrolpfropfcopoylmer (MABS), Aromatische Polyester (APE), Polyestercarbonate (PEC), Cellulosepropionat (CP), Polyetherfluorethylen (PTFE), Poylethersulfon (PES).

12. Vorrichtung zur Herstellung eines Rohrs aus Glas, Kunststoff oder einem Kunststoff-Verbundmaterial mit einem abschnittsweise von einer Kreisform abweichenden Profil durch Umformen, mit
Quetschwalzen (1), die einen Walzenspalt mit einer Spaltbreite ausbilden;
einer den Quetschwalzen zugeordneten Verstelleinrichtung (25), um die Spaltbreite zu verstellen;
einer Steuereinrichtung (88), die der Verstelleinrichtung (25) zugeordnet ist, um diese zu steuern; und
eine Trenneinrichtung (92), um ein Rohr mit einer vorbestimmten Länge abzutrennen;
**dadurch gekennzeichnet, dass** die Steuereinrichtung (88) ausgelegt ist, um ein Verfahren nach einem der vorhergehenden Ansprüche auszuführen, sodass Endabschnitte (L1; L1*) des Rohrs jeweils ein kreisförmiges Profil aufweisen.

13. Vorrichtung nach Anspruch 12, weiterhin umfassend eine Messeinrichtung (91), um eine geförderte Rohrlänge des Rohrs (81) zu messen, wobei die Steuereinrichtung (88) die Verstelleinrichtung (25) ferner auf Grundlage eines Messwerts für die jeweils geförderte Rohrlänge des Rohrs (81) so steuert, dass eine axiale Länge des von der Kreisform abweichenden Profils (L3) und/oder eine axiale Länge von Übergangsbereichen (L2; L2*) zwischen den Endabschnitten (L1; L1*) des Rohrs und dem von der Kreisform abweichenden Profil (L3) eingestellt wird.

14. Vorrichtung nach Anspruch 12 oder 13, wobei die den Quetschwalzen (1) zugeordnete Verstelleinrichtung (25) ferner so ausgelegt oder durch die Steuereinrichtung (88) gesteuert ist, dass die Lage der jeweiligen Quetschwalze (1) durch permanentes axiales Verstellen der jeweiligen Quetschwalze verändert wird, wobei das permanente axiale Verstellen der jeweiligen Quetschwalze entsprechend einer vorbestimmten Bewegungsfunktion erfolgt, wobei die vorbestimmte Bewegungsfunktion
eine zyklische Hin- und Herbewegung der zumindest einen Quetschwalze in deren axialer Richtung ist und/oder
in diskreten Schritten gleicher Schrittgröße ausgeführt wird.

## Claims

1. A method for the production of a tube having, in sections, a non-circular profile by deforming, comprising:
a) providing a tube (81) made of glass, plastic or a plastic composite material, wherein the tube has a circular initial profile (L1);
b) conveying the tube (81) in a hot, malleable state through a nip, which is formed by squeezing rollers (1) and has a first nip width, which is larger than or equal to an outer dimension of the initial profile;
c) adjusting the squeezing rollers (1) for setting a second nip width, which is smaller than the outer dimension of the initial profile, and deforming the initial profile in said hot, malleable state for obtaining said non-circular cross section (L3); and
d) adjusting the squeezing rollers (1) for setting a third nip width, which is larger than or equal to the outer dimension of the initial profile, and severing said tube in a region having a circular cross section;
so that respective end portions (L1; L1*) of said tube have a circular cross section.

2. The method according to claim 1, wherein a conveying length of the tube (81) is measured, wherein an axial length of a section (L3) having said non-circular cross section and/or an axial length of transition portions (L2; L2*) between the end portions (L1; L1*) of the tube and said non-circular profile (L3) is/are adjusted on the basis of a value for the respective conveying length.

3. The method according to claim 2, wherein the adjusting of the squeezing rollers (1) in steps c) and d) is performed according to a predetermined adjustment function for forming the transition portions (L2; L2*) between the end portions (L1; L1*) of the tube and said non-circular profile (L3) such that these transition portions have cross sections in accordance with the predetermined adjustment function.

4. The method according to any of the preceding claims, wherein the deforming of the initial profile (L3) to said non-circular profile in step c) is performed in a hot, malleable state, wherein the position of at least one of the squeezing rollers (1) is continuously varied, in particular by a continuous axial adjustment, so that a contact area between the respective squeezing roller and the hot tube running into said nip is continuously varied.

5. The method according to claim 4, wherein the continuous axial adjustment of the respective squeezing roller is performed in accordance with a predetermined adjustment function, wherein the predetermined adjustment function
is a cyclic reciprocating movement of the respective squeezing roller in an axial direction thereof, and/or
is performed in discrete steps, each having the same step size.

6. The method according to any of the preceding claims, wherein the axes of the squeezing rollers (1), which form the nip taper in a V-shaped manner.

7. The method according to any of the preceding claims, wherein the squeezing rollers (1), which form said nip, have a non-circular profile, in particular an oval or a polygonal profile.

8. The method according to any of the preceding claims, wherein the squeezing rollers (1), which form the nip, comprise a rotational symmetric profile with a contour different from the line shape, wherein the squeezing rollers form in particular a mirror-symmetric nip, and wherein the contour of the respective squeezing roller comprises at least one recess or at least one projection.

9. The method according to any of the preceding claims, wherein the squeezing rollers (1) are driven actively and/or are heated additionally, and/or
wherein the squeezing rollers (1) are adjusted in axial direction and/or an angle of inclination is varied.

10. The method according to any of the preceding claims, wherein the tube (81) having the circular initial profile (L1) is made of a glass, in particular of a transparent glass.

11. The method according to any of claims 1 to 9, wherein the tube (81) having the circular initial profile (L1) is made of a plastic material, in particular of a transparent plastic material, wherein the plastic material is selected from a group consisting of: polymethylmethacrylate (PMMA), polycarbonate (PC), polyamide (PA), polyethylene (PE) polypropylene (PP), polystyrol (PS), poly-4-methylpentene-1 (PMP), polyvinylchloride (PVC), cycloolefincopolymer (COC), styrol/butadien/styrol-bolckcopolymer (SBS), methylmethacrylate/acrylnitrile/polybutadiene/styrolpfropfcopoylmere (MABS), aromatic polyesters (APE), polyestercarbonate (PEC), cellulose propionate (CP), polyetherfluorethylene (PTFE), poylethersulfone (PES).

12. An apparatus for the production of a tube made of glass, plastic or a plastic composite material having, in sections, a non-circular profile by deforming, comprising
squeezing rollers (1), which form a nip having a nip width;
an adjusting device (25) associated to said squeezing rollers for adjusting the nip width;
a controlling device (88) associated to said adjusting device (25) for controlling said adjusting device; and
a severing device (92) for severing a tube having a predetermined length;
**characterized in that** said controlling device (88) is configured, in order to perform the method according to any of the preceding claims so that end portions (L1; L1*) of said tube respectively have a circular profile.

13. The apparatus according to claim 12, further comprising a measuring device (91) for measuring a conveying length of the tube (81), wherein said controlling device (88) controls the adjusting device (25) additionally on the basis of a value for the respective conveying length of the tube such that an axial length of a section (L3) having said non-circular cross section and/or an axial length of transition portions (L2; L2*) between the end portions (L1; L1*) of the tube and said non-circular profile (L3) is/are adjusted on the basis of a value for the respective conveying length.

14. The apparatus according to claim 12 or 13, wherein the adjusting device (25) associated with said squeezing rollers (1) is configured or controlled by the controlling device (88) such that the position of the respective squeezing roller (1) is varied by a continuous axial adjustment of the respective squeezing roller wherein the continuous axial adjustment of the respective squeezing roller is performed in accordance with a predetermined adjustment function, wherein said predetermined adjustment function
is a cyclic reciprocating movement of the at least one respective squeezing roller in an axial direction thereof and/or
is performed in discrete steps, each having the same step size.

## Revendications

1. Procédé de production d'un tube ayant, par parties, un profil de forme non circulaire généré par déformation, comprenant :
a) la mise à disposition d'un tube (81) en verre, en plastique ou en matériau composite plastique, le tube présentant un profil initial circulaire (L1);
b) le déplacement du tube (81) dans un état malléable, à chaud, au travers zone de pincement, qui est délimitée par des rouleaux pinceurs (1) et ayant une première largeur de zone de pincement qui est supérieure ou égale à une dimension extérieure du profil initial ;
c) le réglage des rouleaux de pincement (1) pour définir une seconde zone de pincement, qui est inférieure à la dimension extérieure du profil initial, et la déformation du profil initial dans l'état malléable, à chaud, en vue de l'obtention de ladite section non circulaire (L3) ; et
d) le réglage des rouleaux de pincement (1) pour définir une troisième zone de pincement, qui est supérieure ou égale à la dimension extérieure du profil initial, et à séparer le tube dans une zone ayant une section circulaire;
de sorte que les extrémités terminales respectives (L1, L1*) dudit tube présentent une section circulaire.

2. Le procédé selon la revendication 1, dans lequel est mesurée une longueur de transport du tube (81), avec une longueur axiale d'une partie (L3) ayant ladite section non circulaire et/ou une longueur axiale des zones de transition (L2, L2 *) entre les extrémités terminales (L1, L1 *) du tube et le profil de forme non-circulaire (L3) est/son réglé(s) sur la base d'une valeur de longueur de transport respective.

3. Le procédé selon la revendication 2, dans lequel le réglage des rouleaux de pincement (1) dans les étapes c) et d) est effectué suivant une fonction de réglage prédéterminée pour la formation des zones de transition (L2, L2 *) entre les extrémités terminales (L1, L1 *) du tube et ledit profil non circulaire (L3) de telle sorte que ces zones de transition présentent des sections conformes à la fonction de réglage prédéterminée.

4. Le procédé selon l'une quelconque des revendications précédentes, dans lequel la déformation du profil initial (L3) vers ledit profil non circulaire de l'étape c) est effectuée dans un état malléable, à chaud, dans lequel la position d'au moins un rouleau de pincement (1) est continuellement modifiée, en particulier suivant un ajustement axial continu, de telle sorte que varie continuellement un espace de contact entre le rouleau de pincement et le tube chaud de déplaçant dans ladite zone de pincement.

5. Le procédé selon la revendication 4, dans lequel le réglage axial continu du rouleau de pincement respectif est réalisé suivant une fonction de réglage prédéterminée, dans lequel la fonction de réglage prédéterminée
consiste en un mouvement réciproque cyclique du rouleau de pincement respectif suivant une direction axiale, et/ou
est réalisée par pas discrets, de dimension constante.

6. Le procédé selon l'une quelconque des revendications précédentes, dans lequel les axes des rouleaux de pincement (1) forment la zone de pincement suivant une forme en V.

7. Le procédé selon l'une quelconque des revendications précédentes, dans lequel les rouleaux de pincement (1) qui forment ladite zone de pincement, présentent une forme non circulaire, en particulier une forme ovale ou un polygone.

8. Le procédé selon l'une quelconque des revendications précédentes, dans lequel les rouleaux de pincement (1) qui forment la zone de pincement, comportent un profil à symétrie de rotation avec un contour non rectiligne, dans lequel les rouleaux de pincement forment en particulier une fente à symétrie de miroir, et dans lequel le contour du rouleau de pincement respectif comporte au moins un évidement ou au moins une saillie.

9. Le procédé selon l'une quelconque des revendications précédentes, dans lequel les rouleaux de pincement (1) sont entraînés activement et/ou en outre chauffés, et/ou
dans lequel les rouleaux de pincement (1) sont réglés suivant une direction axiale et/ou avec variation d'un angle d'inclinaison.

10. Le procédé selon l'une quelconque des revendications précédentes, dans lequel le tube (81) présentant le profil initial circulaire (L1) est réalisé en verre, en particulier en verre transparent.

11. Le procédé selon l'une des revendications 1 à 9, dans lequel le tube (81) ayant le profil initial circulaire (L1) est réalisé en matière plastique, en particulier en une matériau plastique transparent, dans lequel le matériau plastique est choisi au sein d'un groupe composé de : méthacrylate de polyméthyle (PMMA ), le polycarbonate (PC), polyamide (PA), polyéthylène (PE), le polypropylène (PP), polystyrène (PS), le poly-4-méthylpentène-1 (PMP), le chlorure de polyvinyle (PVC), les copolymères de cyclooléfine (COC), le styrène/butadiène/styrène-Bolckcopolymère (SBS), le méthacrylate de méthyle/acrylonitrile/polybutadiène /Styrolpfropfcopolymère (MABS), polyester aromatique (APE), carbonate polyester (PEC), le propionate de cellulose (CP), le Polyétherfluoréthylène (PTFE), le Polyléthersulfone (PSE).

12. Un appareil pour produire un tube réalisé en verre, en plastique ou en matériau plastique composite ayant, par sections, un profil non circulaire par déformation, comprenant:
des rouleaux de pincement (1), qui forment une zone de pincement ayant une largeur de pincement ;
un dispositif de réglage (25) associé audits rouleaux de pincement pour régler la largeur de pincement ;
un dispositif de commande (88) associé audit dispositif de réglage (25) pour commander ledit dispositif de réglage : et
un dispositif de séparation (92) pour séparer un tube ayant une longueur prédéterminée ;
**caractérisé en ce que** ledit dispositif de commande (88) est configuré pour mettre en oeuvre le procédé selon l'une quelconque des revendications précédentes en sorte que les extrémités (L1, L1 *) du tube présentent respectivement un profil circulaire.

13. Appareil selon la revendication 12, comprenant en outre un dispositif de mesure (91) pour mesurer une longueur de déplacement du tube (81), dans lequel lesdits moyens de commande (88) commandent le dispositif de réglage (25), en outre sur la base d'une valeur pour chaque longueur de transport du tube (81) de telle sorte qu'une longueur axiale d'une partie (L3) ayant une section non circulaire et/ou une longueur axiale des zones de transition (L2; L2 *) entre les extrémités (L1, L1 *) du tube et ledit profil non circulaire (L3) est/sont réglé(s) sur la base d'une valeur de la longueur de déplacement respective.

14. L'appareil selon la revendication 12 ou 13, dans lequel le dispositif de réglage (25) associé audits rouleaux de pincement (1) est configuré ou commandé par le dispositif de commande (88) de telle sorte que la position du rouleau de pincement respectif varie continuellement suivant un réglage axial du rouleau de pincement respectif, dans lequel le réglage axial continu du rouleau de pincement respectif est effectué suivant une fonction de réglage prédéterminée, dans lequel ladite fonction de réglage prédéterminée :
consiste en un mouvement réciproque cyclique du rouleau de pincement respectif suivant une direction axiale, et/ou
est réalisée par pas discrets, de dimension constante.
